(19) Europäisches Patentamt
European Patent Office
Office européen des brevets

(11) Publication number: **0 283 271 A2**

(12) **EUROPEAN PATENT APPLICATION**

(21) Application number: **88302306.1**

(22) Date of filing: **16.03.88**

(51) Int. Cl.⁴: **C 07 D 237/14**
C 07 D 237/18, A 01 N 43/58

(30) Priority: **17.03.87 JP 60045/87**
**28.10.87 JP 270382/87**

(43) Date of publication of application:
**21.09.88 Bulletin 88/38**

(84) Designated Contracting States: **ES FR GB**

(71) Applicant: **UBE INDUSTRIES, LTD.**
**12-32, Nishihonmachi 1-chome**
**Ube-shi, Yamaguchi-ken 755 (JP)**

**RIKAGAKU KENKYUSHO**
**2-1 Hirosawa**
**Wako-shi Saitama-ken (JP)**

(72) Inventor: **Yoshioka, Hirosuke**
**c/o Rikagaku Kenkyusho 2-1, Hirosawa**
**Wako-shi Saitama-ken (JP)**

**Obata, Tokio**
**c/o Ube Research Laboratory Ube Ind. Ltd. 1978-5**
**Oaza Kogushi Ube-shi Yamaguchi-ken (JP)**

**Fujii, Katsutoshi**
**c/o Ube Research Laboratory Ube Ind. Ltd. 1978-5**
**Oaza Kogushi Ube-shi Yamaguchi-ken (JP)**

**Fukuda, Yasuhisa**
**c/o Ube Research Laboratory Ube Ind. Ltd. 1978-5**
**Oaza Kogushi Ube-shi Yamaguchi-ken (JP)**

**Ooka, Akira**
**c/o Ube Research Laboratory Ube Ind. Ltd. 1978-5**
**Oaza Kogushi Ube-shi Yamaguchi-ken (JP)**

(74) Representative: **Harrison, Michael Robert et al**
**Urquhart-Dykes & Lord 91 Wimpole Street**
**London W1M 8AH (GB)**

Claims for the following Contracting State: ES.

(54) **Pyridazinone derivative, process for producing thereof and insecticide, acaricide and fungicide containing said derivative as active ingredient.**

(57) There are disclosed a pyridazinone derivative represented by the formula:

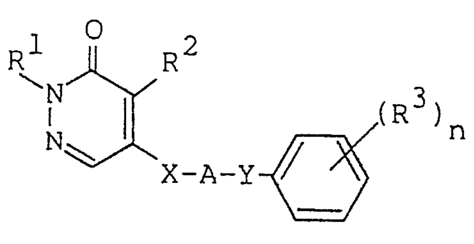

wherein $R^1$ represents a lower alkyl group; $R^2$ represents a halogen atom; $R^3$ represents a hydrogen atom, a halogen atom, a nitro group, a formyl group, a lower alkenyl group, a substituted or unsubstituted alkyl group having 1 to 10 carbon atoms, a lower alkoxy group, a substituted or unsubstituted 1,3-dioxolan-2-yl group, a lower alkoxyiminoalkyl group, an acyl group or a 1,3-thiooxolan-2-yl group; n represents an integer of 1 to 5; A represents a straight or branched alkylene group having 2 to 4 carbon atoms: and X and Y each independently represent an oxygen atom or a sulfur atom.
or an acid addition salt thereof, a process for producing the same, and an insecticide, an acaricide and a fungicide containing the derivative as the active ingredient.

EP 0 283 271 A2

Description

**Pyridazinone derivative, process for producing thereof and insecticide, acaricide and fungicide containing said derivative as active ingredient**

**BACKGROUND OF THE INVENTION**

This invention relates to a pyridazinone derivative, a process for producing thereof and an insecticide, an acaricide and a fungicide containing said derivative as the active ingredient.

Heretofore, many numbers of pyridazinone derivatives have been known in the art. Among them, as those having fungicidal, insecticidal and acaricidal activities, there have been disclosed 5-phenylalkoxy- or 5-phenylalkylthiopyridazinone derivatives (Japanese Provisional Patent Publications No. 152878/1983, No. 98064/1984, No. 4173/1985, No. 32774/1985 and No. 54370/1985), 5-allyloxy- or 5-allylthiopyridazinone derivatives (Japanese Provisional Patent Publication No. 268672/1986), etc. Further, in Japanese Provisional Patent Publication No. 130275/1986, there have been disclosed pyridazinone derivatives and among these, as a compound having a cross-linking moiety of $-S-CH_2CH_2-O-$, there have been disclosed the compound represented by the following formula:

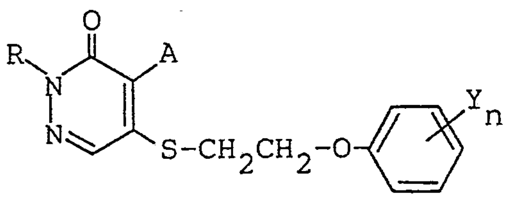

wherein R represents an alkyl group having 1 to 6 carbon atoms; A represents an alkyl group having 1 to 6 carbon atoms; Y represents a hydrogen atom, a halogen atom, an alkyl group having 1 to 6 carbon atoms, a cycloalkyl group, a substituted or unsubstituted alkyloxy group or alkylthio group, etc.; and n is an integer of 1 to 5.

The above known compound has fungicidal, insecticidal and acaricidal activities, and is effective against injurious insects such as green rice leafhopper, 28-spotted lady beetle, etc., Kanzawa spider mite, and also against diseases such as downy mildew, powdery mildew, scab, blast, etc.

However, the compound which is concretely disclosed in the examples is only the compounds represented by the following formula:

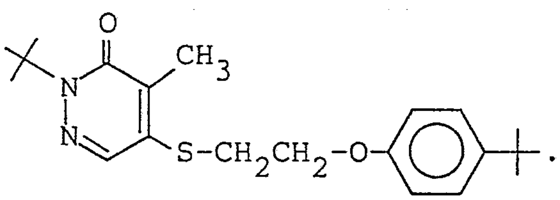

Such a compound is not sufficient in activity, while it has fungicidal, insecticidal and acaricidal activities The present inventors have intensively studied in order to obtain compounds having further excellent insecticidal, acaricidal and fungicidal activities than the known compound as mentioned above, and consequently found that a phenoxyalkylthio pyridazinone derivative in which a halogen atom is incorporated at 4-position of the pyridazinone ring in the above formula, is a compound having remarkably improved insecticidal and acaricidal activities, and accomplished the present invention.

**SUMMARY OF THE INVENTION**

This invention provides a pyridazinone derivative represented by the formula (I):

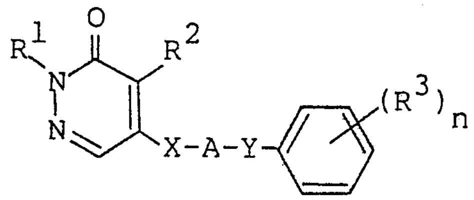

(I)

wherein $R^1$ represents a lower alkyl group; $R^2$ represents a halogen atom; $R^3$ represents a hydrogen atom, a halogen atom, a nitro group, a formyl group, a lower alkenyl group, a substituted or unsubstituted alkyl group having 1 to 10 carbon atoms, a lower alkoxy group, a substituted or unsubstituted 1,3-dioxolan-2-yl group, a lower alkoxyiminoalkyl group, an acyl group or a 1,3-thiooxolan-2-yl group; n is an integer of 1 to 5; A represents a straight or branched alkylene group having 2 to 4 carbon atoms; and X and Y each independently represent an oxygen atom or a sulfur atom;
or an acid addition salt thereof, a process for producing the same, and an insecticide, an acaricide and a fungicide containing the same as an active ingredient.

## DESCRIPTION OF THE PREFERRED EMBODIMENT

In the above formula (I), the lower alkyl group represented by $R^1$ refers to a straight or branched alkyl group having 1 to 5 carbon atoms. Such alkyl groups may include a methyl group, an ethyl group, a propyl group, an isopropyl group, a butyl group, an isobutyl group, a sec-butyl group, a t-butyl group, an amyl group, an isoamyl group, a sec-amyl group, a sec-isoamyl group (a 1,2-dimethylpropyl group), a t-amyl group (a 1,1-dimethylpropyl group), etc.

The lower alkenyl group represented by $R^3$ refers to a straight or branched alkenyl group having 2 to 5 carbon atoms, and may include, for example, a vinyl group, an allyl group, a 1-propenyl group, a methallyl group, etc. Further, an alkyl group having 1 to 10 carbon atoms may include a methyl group, an ethyl group, a propyl group, an isopropyl group, a butyl group, an isobutyl group, a sec-butyl group, a t-butyl group, an amyl group, an isoamyl group, a sec-amyl group, a sec-isoamyl group (a 1,2-dimethylpropyl group), and a t-amyl group (a 1,1-dimethylpropyl group), a hexyl group, a 2-ethylbutyl group, a heptyl group, an octyl group, a 2-ethylhexyl group, a nonyl group, a decyl group, etc.

When $R^3$ is a substituted alkyl group having 1 to 10 carbon atoms, such substituents may include a halogen atom, a hydroxy group, a lower alkenyl group, a lower alkylthio group, a lower alkoxy group, a lower alkoxyalkyloxy group, a lower alkenyloxy group, a lower alkynyloxy group, an aralkyloxy group, a lower alkylsulfonyl group, a lower alkylamino group, a lower alkylthioalkoxy group, a lower haloalkyloxy group, a cycloalkyloxy group, an acyloxy group, a lower alkoxycarbonyl group, a 1,3-dioxolan-2-yl group or a pyranyloxy group.

The lower alkylthio group refers to a straight or branched alkylthio group having 1 to 5 carbon atoms. Such alkylthio groups may include a methylthio group, an ethylthio group, a propylthio group, an isopropylthio group, a butylthio group, an isobutylthio group, a sec-butylthio group, a t-butylthio group and an amylthio group.

The lower alkoxy group refers to a straight or branched alkoxy group having 1 to 5 carbon atoms Such alkoxy groups may include a methoxy group, an ethoxy group, a propoxy group, an isopropoxy group, a butoxy group, an isobutoxy group, a sec-butoxy group, a t-butoxy group, an amyloxy group, etc.

The lower alkenyloxy group may include an allyloxy group, a propenyloxy group, etc.

The lower alkynyloxy group may include a propargyloxy group, a 1-propynyloxy group, etc.

The lower alkoxyalkyloxy group means a lower alkoxy group substituted by the above mentioned lower alkoxy group, and may include, for example, a methoxymethyloxy group, a 1-or 2-methoxyethyloxy group, a 1- or 2-ethoxyethyloxy group, etc.

The aralkyloxy group may include a benzyloxy group, a 1-phenethyloxy group, a 2-phenethyloxy group, etc.

The lower alkylsulfonyl group refers to a straight or branched alkylsulfonyl group having 1 to 4 carbon atoms. Such alkylsulfonyl group may include a methanesulfonyl group, an ethanesulfonyl group, a propanesulfonyl group, etc.

The lower alkylamino group refers to a straight or branched alkylamino group having 1 to 4 carbon atoms. Such alkylamino group may include a methylamino group, an ethylamino group, a propylamino group, an isopropylamino group, a butylamino group, an isobutylamino group, a sec-butylamino group and t-butylamino group.

The lower alkylthioalkyl group represents an alkoxy group having 1 to 4 carbon atoms which is substituted by the above mentioned alkylthio group, and may include, for example, a methylthiomethyloxy group, an ethylthiomethyl oxy group, a methylthioethyloxy group or an ethylthioethyloxy group.

The lower haloalkyloxy group refers to a straight or branched alkyloxy group having 1 to 4 carbon atoms which is substituted by 1 to 3 halogen atoms Such lower halo alkyloxy group may include a 2-chloroethyloxy group, a 2-bromoethyloxy group, a 2-fluoroethyloxy group, a 2,2,2-trifluoroethyloxy group, etc.

The cycroalkyloxy group refers to a cycloalkyloxy group having 3 to 6 carbon atoms Such cycloalkyloxy

group may include a cyclopropyloxy group, a cyclopentyloxy group, a cyclohexyloxy group, etc.

The acyloxy group may include an acetyloxy group, a propionyloxy group, a pivaloyloxy group, a 2-chlorobenzoyloxy group, etc.

The lower alkoxycarbonyl group represents a carbonyloxy group substituted by the above-mentioned alkoxy group. Such lower alkoxycarbonyl group may include a methoxycarbonyl group, an ethoxycarbonyl group, a propoxycarbonyl group, an isopropoxycarbonyl group and a butoxycarbonyl group.

When $R^3$ is a lower alkoxy group, a substituted or unsubstituted 1,3-dioxolan-2-yl group, a lower alkoxyiminoalkyl group or an acyl group, each represents the following substituent:

The lower alkoxy group refers to a straight or branched alkoxy group having 1 to 5 carbon atoms. Such alkoxy group may include a methoxy group, an ethoxy group, a propoxy group, an isopropoxy group, etc.

The substituted or unsubstituted 1,3-dioxolan-2-yl group refers to a 1,3-dioxolan-2-yl group which may be substituted by 1 or 2 alkyl groups or by 1 or 2 alkenyloxymethyl groups. Such substituted 1,3-dioxolan-2-yl group may include a 4-methyl-1,3-dioxolan-2-yl group, a 4,5-dimethyl-1,3-dioxolan-2-yl group, a 4-allyloxymethyl-1,3-dioxolan-2-yl group, etc.

The lower alkoxyiminoalkyl group may include a methoxyiminomethyl group, a 2-ethoxyiminoethyl group, a 1-methoxyiminopentyl group, etc.

The acyl group may include an acetyl group, a propionyl group and a valeroyl group, etc.

$R^1$ may preferably be a tert-butyl group.

$R^2$ may preferably be a chlorine atom or a bromine atom.

As the $R^3$, at least one of $R^3$ may preferably be at 4-position of the benzene ring, and such substituent $R^3$ may preferably be a propyl group, a butyl group, a pentyl group, a 2,2-difluoropropyl group, a 2-ethylthioethyl group, an ethoxymethyl group, a 1-methoxyethyl group, a 2-methoxyethyl group, a 2-ethoxyethyl group, a 2-propoxyethyl group, a 2-butoxyethyl group, a 2-allyloxyethyl group, a 2-propargyloxyethyl group, a 2-benzyloxyethyl group, a 2-methoxypropyl group, a 2-methoxybutyl group, a 4-methoxybutyl group, a 2-(2-ethoxyethyloxy)ethyl group, a 2-(1-ethoxyethyloxy)ethyl group and a 1,3-dioxolan-2-ylmethyl group. Further, the other substituent may prefer ably be a hydrogen atom, a methyl group, an ethyl group, a propyl group, a chlorine atom, a bromine atom at the same time.

n may preferably be 2 or 3.

A may preferably be an ethylene group.

X may preferably be an oxygen atom or a sulfur atom.

Y may preferably be an oxygen atom.

In the above formula (I), when any of carbon atoms is an asymmetric carbon atom, its optical isomers and racemic compound or mixture thereof are all included in the present invention.

The compound (I) of the present invention can be easily prepared according to the process shown below, which are known *per se* in the art.

Preparation process (1)

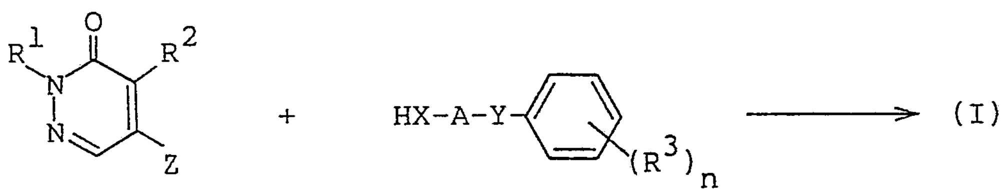

(II) (III)

wherein $R^1$, $R^2$, $R^3$, n, A, X and Y are as defined above; and Z represents a halogen atom.

Preparation process (2)

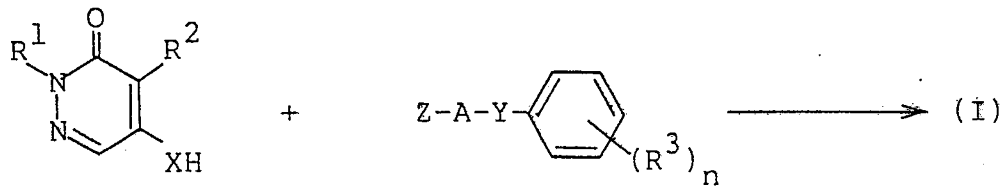

(II) (V)

wherein $R^1$, $R^2$, $R^3$, n, A, X, Y and Z are as defined above.

Preparation process (3)(the case where $R^3$ is an alkylaminoalkyl group)

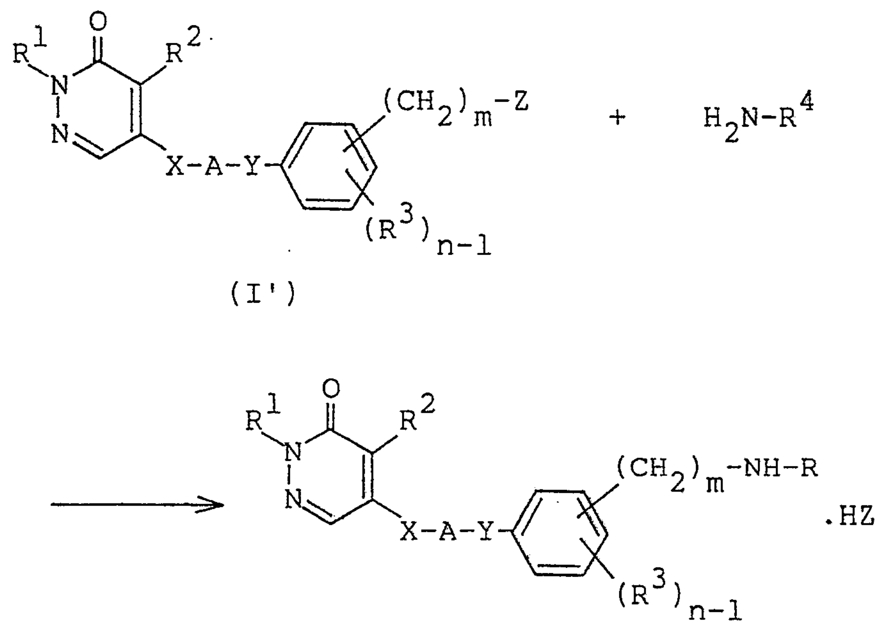

(I')

(I)

wherein $R^1$, $R^2$, $R^3$, n, A, X and Y are as defined above; Z represents a halogen atom; $R^4$ represents a straight or branched alkyl group having 1 to 4 carbon atoms; and m is 1 or 2.

Preparation process (4)(the case where $R^3$ is an alkoxyliminoalkyl group)

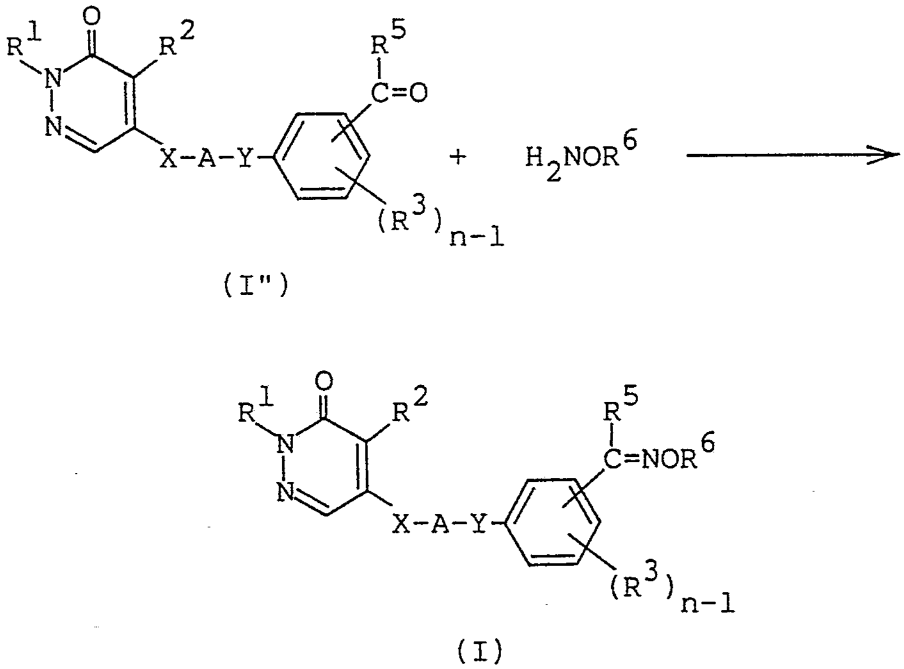

(I")

(I)

wherein $R^1$, $R^2$, $R^3$, n, A, X and Y are as defined above; and $R^5$ and $R^6$ each represent a straight or branched alkyl group having 1 to 5 carbon atoms.

Preparation process (5)(the case where $R^3$ is a substituted or unsubstituted 1,3-dioxolan-2-yl group or 1,3-thiooxolan-2-yl group)

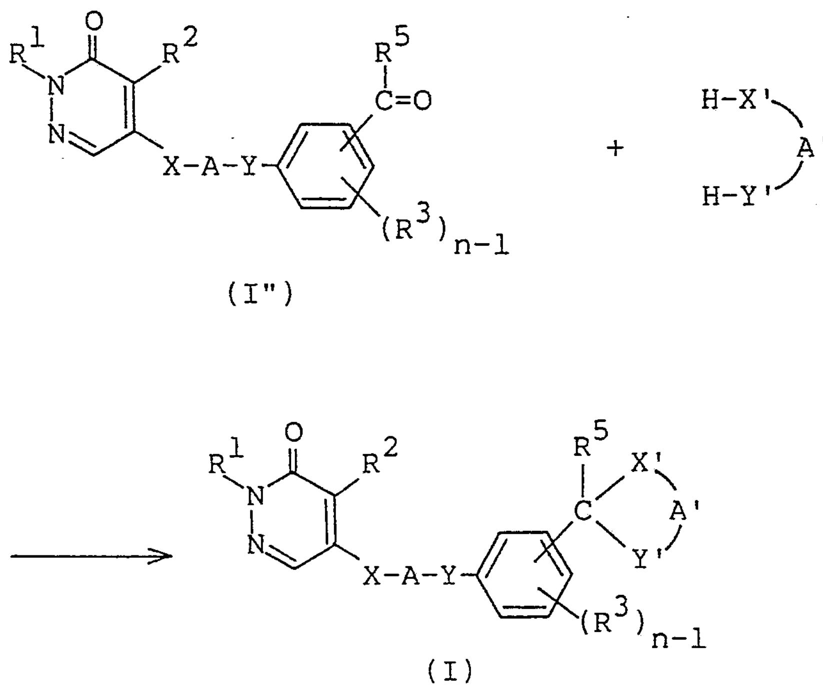

(I")

(I)

wherein $R^1$, $R^2$, $R^3$, n, A, X and Y are as defined above; $R^5$ represents a straight or branched alkyl group having 1 to 5 carbon atoms; X′ and Y′ each independently represent an oxygen atom or a sulfur atom; and A′ represents an alkylene group having 2 or 3 carbon atoms which may be substituted by an alkyl group or an

alkenyloxymethyl group.

Preparation process (6)(the case where $R^3$ is an acyloxyalkyl group)

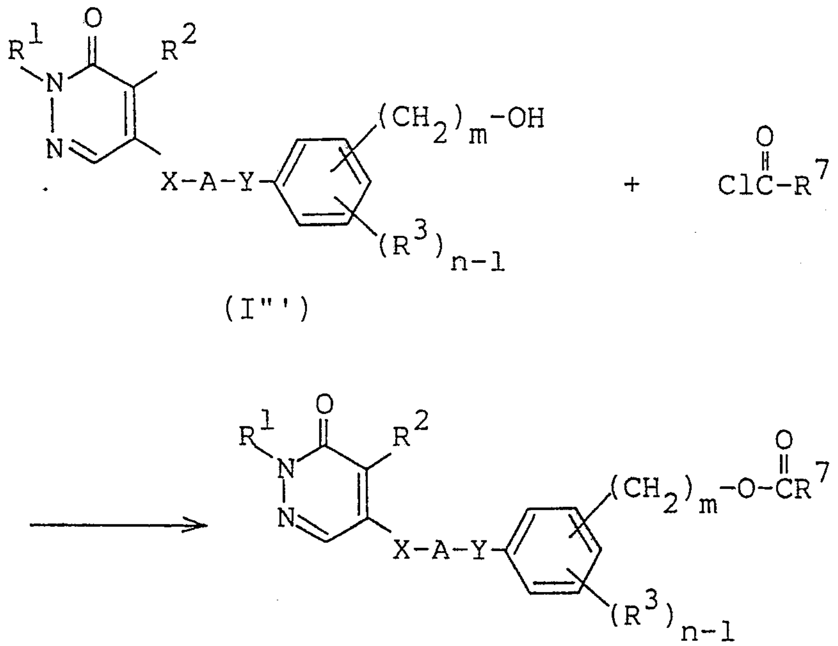

(I"')

(I)

wherein $R^1$, $R^2$, $R^3$, n, A, X and Y are as defined above; $R^7$ represents a straight or branched alkyl group having 1 to 5 carbon atoms or a 2-chlorophenyl group; and m is 1 or 2.

As clearly seen from the reaction schemes shown above, a halogenated hydrogen (HX) is eliminated after the reaction in any cases of the preparation processes 1 or 2. Therefore, for capturing HX and allowing the reaction to proceed smoothly, it is preferred to carry out the reaction in the presence of a base.

The base may include organic bases such as triethylamine, pyridine and N,N-dimethylaniline; alkali metal alkoxides such as sodium methoxide and sodium ethoxide; inorganic bases such as sodium hydride, sodium amide, sodium hydroxide, potassium hydroxide, sodium carbonate, sodium hydrogencarbonate and potassium carbonate; etc.

The reaction is generally carried out in the presence of a solvent. The solvent is not particularly limited as long as it does not participate in the above reaction and may include aromatic, aliphatic or alicyclic hydrocarbons which are chlorinated or not chlorinated, such as benzene, toluene, xylene, methylnaphthalene, petroleum ether, ligroin, hexane, chlorobenzene, dichlorobenzene, methylene chloride, chloroform, dichloroethane, trichloroethylene and cyclohexane; ethers such as diethyl ether, ethylene glycol dimethyl ether, tetrahydrofuran and dioxane; ketones such as acetone and methyl ethyl ketone; alcohols such as methanol, ethanol and ethylene glycol, or hydrates thereof; amides such as N,N-dimethylformamide; water and a mixture of the solvents described above; etc.

However, when inorganic bases such as sodium hydroxide, potassium hydroxide are used as the above mentioned bases, it is preferred to use water or a mixture of the above solvents and water in order to dissolve these bases.

Alternatively, when a mixture of water and a non hydrophilic solvent is used, it is preferable to add a small quantity of phase transfer catalyst. Such phase transfer catalysts may include quarternary ammonium salts such as tetramethylammonium chloride, triethylbenzylammonium chloride, tetra-n-butylammonium bromide or tri-n-octylmethylammonium chloride; phosphonium salts such as tetra-n-butylphosphonium bromide; crown ethers such as dicyclohexano-18-crown-6; cryptands; etc.

The reaction temperature is not particularly limited but generally from 0 °C to the boiling point of the solvent used. Heating should preferably be carried out for shortening the reaction time.

The compounds represented by the formulae (II), (III), (IV) and (V), which are used as the feedstocks in the above mentioned preparation processes, can be easily prepared according to the process shown below, which are known *per se* in the art.

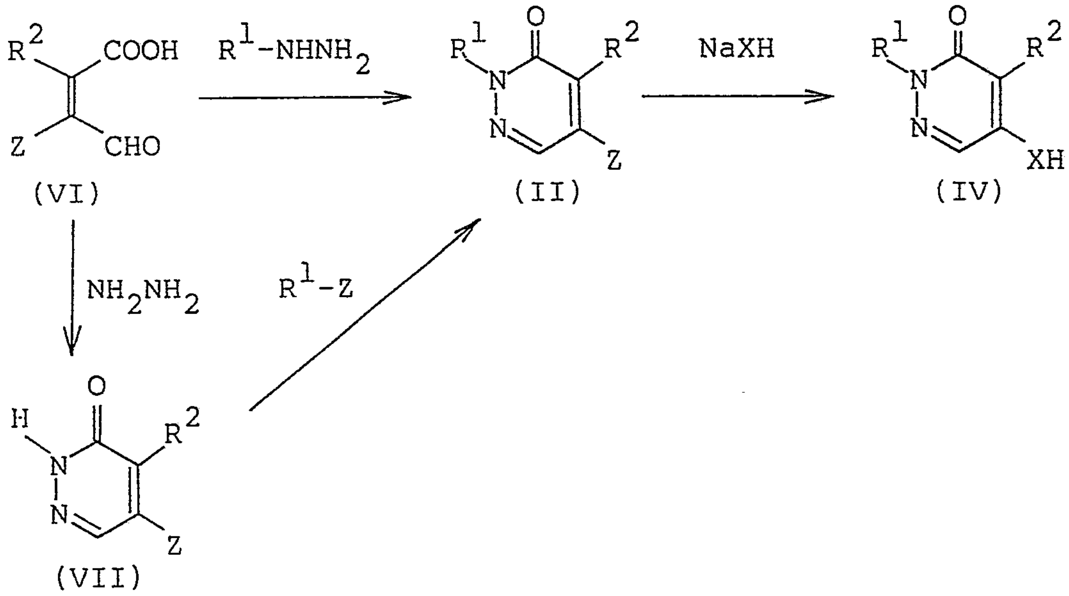

(VI) (II) (IV) (VII)

wherein $R^1$, $R^2$, X and Z are as defined above.

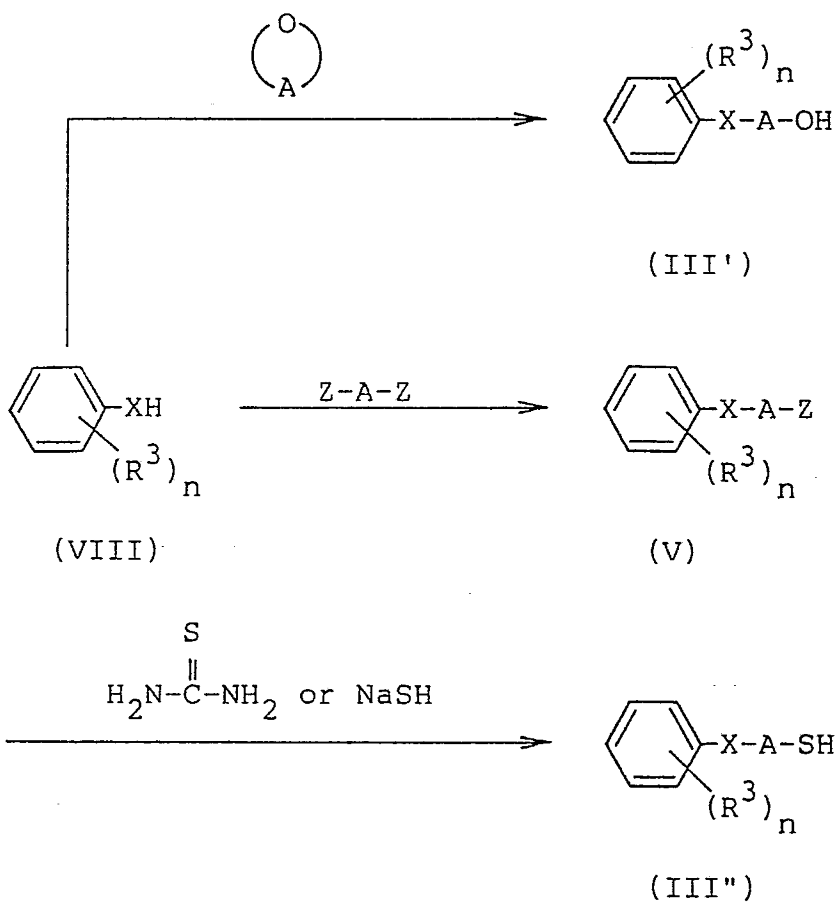

(III') (VIII) (V) (III")

wherein $R^3$, n, A, X, Y and Z are as defined above.

The desired product (I) obtained according to the processes as described above can be purified suitably by known means such as recrystallization, various chromatographies, etc.

The compounds of the present invention exhibit excellent effects against Hemiptera such as planthoppers,

leafhoppers, aphids, white flies, etc.; Leipdoptera such as cabbage armworms, diamondback moth, leaf roller moths, pyralid moths, common white, etc.; Coleoptera such as weevils, leaf beetles, etc.; and other agricultural and horticultural injurious insects, for example, Acarina such as citrus red mite, two-spotted spider mite, etc. Also, they are very effective for prevention and extermination of hygienically injurious insects such as fly, mosquito, cockroach, etc., and also effective for other injurious insects against stored crops.

Further, the compounds of the present invention have also activities against root-knot nematode, pine wood nematode, bulb mite in the soil. Also, the compounds of the present invention are effective and active against diseases for agriculture and horticulture such as blast, powdery mildew, and otherwise downy mildew, gray mold, etc.

Thus, the uses and application fields of the compounds of the present invention are very wide, and therefore they can be provided for practical application in various dosage forms with high activities.

The insecticide, the acaricide and the fungicide of the present invention may comprise one or several kinds of the compounds of the formula (I) as active ingredients. Although the compound of the formula (I) may be used *per se*, it is generally formulated with common carriers, surfactants, dispersing agents or auxiliary agents, and prepared in a conventional manner into compositions such as powder, wettable powder, emulsion, fine granule, granule, aqueous or oily suspension, aerosol, etc. before use.

Suitable carriers may include, for example, solid carriers such as talc bentonite, clay, kaolin, diatomaceous earth, white carbon, vermicullite, slaked lime, siliceous sand, ammonium sulfate, urea, etc.; liquid carriers, for example, hydrocarbons such as kerosene, mineral oil, etc.; aromatic hydrocarbons such as benzene, toluene, xylene, etc.; chlorinated hydrocarbons such as chloroform, carbon tetrachloride, etc.; ethers such as dioxane, tetrahydrofuran, etc.; ketones such as acetone, cyclohexanone, isophorone, etc.; esters such as ethyl acetate, ethylene glycol acetate, dibutyl maleate, etc.; alcohols such as methanol, n-hexanol, ethylene glycol, etc.; polar solvents such as dimethylformamide, dimethyl sulfoxide, etc.; or water. Also, as gaseous carrier, air, nitrogen, carbon dioxide, Freon, etc. can be used to effect mixed jetting.

Also, as the surfactant or the dispersing agent for improvement of attachment or absorption of the present agent onto animals and vegetables or for improvement of performances of dispersion, emulsification or spreading of the medicine, there may be employed, for example, alcohol sulfate esters, alkyl sulfonate salts, lignin sulfonate salts polyoxyethylene glycol ethers, etc.

Further, for amelioration of the properties of the preparation, as the auxiliary agent, for example, carboxymethyl cellulose, polyethylene glycol, gum arabic, etc may be employed.

The above carriers, surfactants, dispersing agents and auxiliary agents may be used either individually or in combination depending on the respective purposes.

The active ingredient concentration when the compound of the present invention is formed into a preparation may be generally 1 to 50 % by weight for emulsion, generally 0.3 to 25 % by weight for powder, generally 1 to 90 % by weight for wettable powder, generally 0.5 to 5 % by weight for granule, generally 0.5 to 5 % by weight for oil and generally 0.1 to 5 % by weight for aerosol.

These preparations can be diluted to appropriate concentrations, and provided for various uses depending on the respective purposes, such as by spraying onto stalks or leaves of vegetables, the surface of soil or paddy field, or alternatively by direct application.

## EXAMPLES

The present invention is described below in more detail by referring to Examples, by which the present invention is not limited at all.

### Example 1

Synthesis of 2-methyl-4-chloro-5-[2-(2-methyl-4-n-pentylphenoxy)ethylthio]-3(2H)-pyridazinone (Compound No. 1)

In methanol were dissolved 1.8 g of 2-methyl-4,5-dichloro-3(2H)-pyridazione and 2.4 g of 2-(2-methyl-4-n-pentylphenoxy)ethylmercaptan and 1 ml of a 28 % solution of sodium methoxide dissolved in methanol was added dripwise to the solution while cooling below 5 °C and stirring. After dropwise addition, the mixture was stirred at room temperature for 1 hour to complete the reaction.

Then, the reaction mixture was poured into water, and crystals precipitated were filtered. After the precipitates were washed with water and dried, they were recrystallized from toluene-n-hexane (1 : 9) to give 2.8 g of the title compound as colorless powdery crystals.
m.p.: 99 to 101 °C.

### Example 2

Synthesis of 2-tert-butyl-4-chloro-5[4-(2-methyl-4-n-propylphenoxy)butylthio]-3(2H)-pyridazinone (Compound No. 7)

In 20 ml of water were added 2.2 g of 2-tert-butyl-4,5-di chloro-3(2H)-pyridazinone, 0.9 g of a 70 % (purity) sodium hydrogensulfide and 0.4 g of sodium hydroxide, and the mixture was stirred at 40 to 60 °C for 30 minute.

Then, to the mixture were added a solution of 2.9 g of 4-(2-methyl-4-n-propylphenoxy)butylbromide

dissolved in 20 ml of benzene and 0.05 g of triethylbenzylammonium chloride, and the mixture was stirred at 40 to 50 °C for 4 hours.

After completion of the reaction, the reaction solution was extracted with toluene. The extract was washed with a 5 % aqueous sodium hydroxide and then with water, dried over anhydrous magnesium sulfate, and then toluene was distilled off under reduced pressure.

The oily product obtained was isolated by column chromatography (Wako Gel C-200, eluted with toluene : ethyl acetate = 9 : 1) to give 1.8 g of the title compound as pale yellow oily liquid.

$n_D^{29.0}$ 1.5484

Example 3

Synthesis of 2-tert-butyl-4-chloro-5-[2-(2-methyl-4-n-pentylphenoxy)ethylthio]-3(2H)-pyridazinone (Compound No. 12)

In 20 ml of methanol were dissolved 1.8 g of 2-tert-butyl-4,5-dichloro-3(2H)-pyridazinone and 1.9 g of 2-(2-methyl-4-n-pentylphenoxy)ethylmercaptan, and then to the mixture was added dropwise a solution of 0.5 g of sodium methoxide dissolved in 5 ml of methanol. After dropwise addition, the mixture was stirred at room temperature for 1 hour to complete the reaction.

Then, methanol was distilled off under reduced pressure, and an oily product which was separated by adding water was extracted with ethyl acetate The extract was washed with water, dried over anhydrous sodium sulfate, and then ethyl acetate was distilled off under reduced pressure. The oily product obtained was isolated by column chromatography (Wako Gel C-200, eluted with toluene : ethyl acetate = 9 : 1) to give 2.8 g of the title compound as colorless oily liquid.

$n_D^{29.0}$ 1.5463

Example 4

Synthesis of 2-tert-butyl-4-chloro-5-[2-{2-methyl-4-(2-methoxyethyl)phenoxy}ethylthio]-3(2H)-pyridazinone (Compound No. 17)

In 30 ml of water were dissolved 2.2 g of 2-tert-butyl-4-chloro-5-mercapto-3(2H)-pyridazinone and 0.5 g of sodium hydroxide.

Then, to the solution were added a solution of 2.7 g of 2-[2-methyl-4-(2-methoxyethyl)phenoxy]-ethylbromide dissolved in 30 ml of toluene and 0.05 g of tetra-n-butylammonium bromide, and the mixture was stirred at 50 to 60 °C for 6 hours.

After completion of the reaction, the reaction solution was extracted with toluene, and the extract was washed with 5 % aqueous sodium hydroxide, and then with water, dried over anhydrous sodium sulfate, and then toluene was distilled off under reduced pressure.

The oily product obtained was isolated by column chromatography (Wako Gel C-200, eluted with toluene : ethyl acetate = 9 : 1) to give 3.9 g of the title compound as pale yellow oily liquid.

$n_D^{18.2}$ 1.5765

Example 5

The procedures were carried out in the same manner as in Examples 1 to 4, to give compounds indicated as Compounds Nos. 2 to 6, 8 to 11, 13 to 16 and 18 to 38 shown in Table 1.

Example 6

Synthesis of 2-tert-butyl-4-chloro-5-[2-{4-(2-methoxyethyl)phenoxy}ethyloxy]-3(2H)-pyridazinone (Compound No. 39)

In 50 ml of N,N-dimethylformamide were dissolved 2.2 g of 2-tert-butyl-4,5-dichloro-3(2H)-pyridazinone and 2.0 g of 2-[4-(2-methoxyethyl)phenoxy]ethanol, 0.8 g of powder of potassium hydroxide was added, and the mixture was stirred at room temperature for 8 hours.

After completion of the reaction, water was added to the solution, a separated oily product was extracted with toluene, the extract was washed with water, dried over anhydrous magnesium sulfate, and then toluene was distilled off under reduced pressure.

The oily product obtained was isolated by column chromatography (Wako Gel C-200, eluted with toluene : ethyl acetate = 9 : 1) to give 2.7 g of the title compound as colorless powdery crystals.

m.p.: 77 to 81 °C

Example 7

Synthesis of 2-tert-butyl-4-chloro-5-[2-{4-(2-ethylthioethyl)phenoxy}ethylthio]-3(2H)-pyridazinone (Compound No. 41)

In 50 ml of toluene were dissolved 2.2 g of 2-tert-butyl-4-chloro-5-mercapto-3(2H)-pyridazinone and 2.9 g of 2-[4-(2-ethylthioethyl)phenoxy]ethylbromide. Then, to the solution were added a solution of 1.2 g of potassium hydroxide dissolved in 20 ml of water and 0.05 g of triethylbenzylammonium chloride, and the mixture was

stirred at 40 to 50 °C for 4 hours.

After completion of the reaction, the toluene layer was separated, washed with water, dried over anhydrous sodium sulfate, and then toluene was distilled off under reduced pressure.

The oily product obtained was isolated by column chromatography (Wako Gel C-200, eluted with toluene : ethyl acetate = 9 : 1) to give 2.5 g of the title compound as pale yellow oily liquid.

$n_D^{25.2}$ 1.5866

Example 8

Synthesis of 2-tert-butyl-4-chloro-5-[2-{4-(2-ethoxyethyl)-2-methylphenoxy}ethyloxy]-3(2H)-pyridazinone (Compound No. 47)

In 50 ml of N,N-dimethylformamide were dissolved 2.0 g of 2-tert-butyl-4-chloro-5-hydroxy-3(2H)-pyridazinone and 2.8 g of 2-[4-(2-ethoxyethyl)-2-methylphenoxy]ethyl bromide, 1.4 g of anhydrous potassium carbonate was added, and the mixture was stirred at 100 to 120 °C for 4 hours.

After completion of the reaction, the reaction solution was poured into water and extracted with toluene. The extract was washed with water, dried over anhydrous magnesium sulfate, and then toluene was distilled off under reduced pressure.

The oily product obtained was isolated by column chromatography (Wako Gel C-200, eluted with toluene : ethyl acetate = 9 : 1) to give 3.0 g of the desired product as colorless oily liquid. $n_D^{24.2}$ 1.5438

Example 9

Synthesis of 2-tert-butyl-4-chloro-5-[2-(2,6-dichloro-4-n-propylphenoxy)ethylthio]-3(2H)-pyridazinone (Compound No. 74)

In 50 ml of N,N-dimethylformamide were dissolved 2.2 g of 2-tert-butyl-4-chloro-5-mercapto-3(2H)-pyridazinone and 3.1 g of 2-(2,6-dichloro-4-n-propylphenoxy)ethyl bromide, to the solution was added 1.4 g of anhydrous potassium carbonate, and the mixture was stirred at 40 to 60 °C for 4 hours.

After completion of the reaction, the reaction solution was poured into water, and a separated oily product was extracted with toluene The extract was washed with water, dried over anhydrous sodium sulfate, and then toluene was distilled off under reduced pressure.

The oily product obtained was isolated by column chromatography (Wako Gel C-200, eluted with toluene : ethyl acetate = 9 : 1) to give 3.2 g of the title compound as pale yellow oily liquid.

$n_D^{23.6}$ 1.5822

Example 10

The procedures were carried out in the same manner as in Examples 6 to 9, to give compounds indicated as Compounds Nos. 40, 42 to 46, 48 to 73 and 75 to 97 shown in Table 1.

Example 11

Synthesis of 2-tert-butyl-4-chloro-5-[2-(2,6-dimethyl-4-tert-butylaminomethylphenoxy)ethylthio]-3(2H)-pyridazinone.hydrochloride (Compound No. 103)

0.5 g of 2-tert-butyl-4-chloro-5-[2-(2,6-dimethyl-4-chloromethylphenoxy)ethylthio]-3(2H)-pyridazinone was mixed with 1 ml of tert-butylamine, and the mixture was stirred at room temperature for 8 hours.

After completion of the reaction, the reaction solution was extracted with toluene, and the extract was washed with water, dried over anhydrous sodium sulfate, and then toluene was distilled off under reduced pressure.

The oily product obtained was isolated by column chromatography (Wako Gel C-200, eluted with toluene : ethyl acetate = 1 : 1) to give 98 mg of the desired product as colorless oily liquid.

$n_D^{24.4}$ 1.5612

Elementary analysis: $C_{23}H_{35}Cl_2N_3O_2S$

| | H | C | N |
|---|---|---|---|
| calculated: | 7.02 | 56.54 | 8.60 |
| found: | 7.53 | 56.58 | 8.50 |

Example 12

Synthesis of 2-tert-butyl-4-chloro-5-[2-(2,6-dichloro-4-N-methoxyiminomethylphenoxy)ethylthio]-3(2H)-pyridazinone (Compound No. 108)

A mixture of 1.2 g of 2-tert-butyl-4-chloro-5-[2-(2,6-dichloro-4-formylphenoxy)ethylthio]-3(2H)-pyridazinone, 0.5 g of O-methylhydroxylamine.hydrochloride, 1 ml of 1N- hydrochloric acid and 20 ml of ethanol was stirred at room temperature for 8 hours.

After completion of the reaction, the reaction solution was extracted with toluene, and the extract was washed with water, dried over anhydrous sodium sulfate, and then toluene was distilled off under reduced pressure.

The oily product obtained was isolated by column chromatography (Wako Gel C-200, eluted with toluene : ethyl acetate = 9 : 1) to give 0.56 g of the desired product as colorless crystals.
m.p.: 81 to 82 °C

Example 13

Synthesis of 2-tert-butyl-4-chloro-5-[2-{2-chloro-4-(1,3-dioxolan-2-yl-methyl)-6-methylphenoxy}ethylthio]-3-(2H)-pyridazinone (Compound No. 114)

In 20 ml of N,N-dimethylformamide were dissolved 0.7 g of 2-tert-butyl-4-chloro-5-mercapto-3-(2H)-pyridazinone and 1 g of 2-chloro-4-(1,3-dioxolan-2-yl-methyl)-6-methylphenoxyethyl bromide, 1 g of anhydrous potassium carbonate was added thereto, and the mixture was stirred at room temperature for 8 hours.

After completion of the reaction, the reaction solution was poured into water and extracted with toluene. The extract was washed with water, dried over anhydrous sodium sulfate, and then toluene was distilled off under reduced pressure.

The oily product obtained was isolated by column chromatography (Wako Gel C-200, eluted with toluene : ethyl acetate = 25 : 1) to give 0.2 g of the title compound as colorless crystals.
m.p.: 82 to 83 °C

Example 14

Synthesis of 2-tert-butyl-4-chloro-5-[2-{2,6-dichloro-4-(1,3-dioxolan-2-yl)phenoxy}ethylthio]-3-(2H)-pyridazinone (Compound No. 104)

A mixture of 0.9 g of 2-tert-butyl-4-chloro-5-[2-(2,6-dichloro-4-formylphenoxy)ethylthio-3-(2H)-pyridazinone, 5 ml of ethyleneglycol, 0.1 g of p-toluenesulfonic hydrate and 50 ml of benzene was refluxed under heating with being attached of water separator for 4 hours, and then the reaction solution was cooled and washed with a diluted aqueous sodium carbonate and then water, dried over anhydrous sodium sulfate, and then the solvent was distilled off under reduced pressure.

The oily product obtained was isolated by column chromatography (Wako Gel C-200, eluted with toluene : ethyl acetate = 35 : 1) to give 0.8 g of the title compound as colorless oily liquid.
$n_D^{26.2}$ 1.5915

Example 15

Synthesis of 2 tert-butyl-4-chloro-5-[2-{2-chloro-4-(2-acetyloxyethyl)-6-methylphenoxy}ethylthio]-3-(2H)-pyridazinone (Compound No. 117)

In 20 ml of dioxane was dissolved 1.2 g of 2-tert-butyl-4-chloro-5-[2-{2-chloro-4-(2-hydroxyethyl)-6-methylphenoxy}ethylthio]-3-(2H)-pyridazinone and then 5 ml of pyridine was added thereto. To the mixture was added dropwise 0.5 g of acetyl chloride under stirring and cooling, and the mixture was stirred for 30 minutes. Then, the mixture was stirred at room temperature for 3 hours to complete the reaction.

The reaction solution was poured into water, and then a separated oily product was extracted with toluene. The extract was washed with a diluted aqueous hydrochloric acid and then with water, dried over anhydrous magnesium sulfate, and then toluene was distilled off under reduced pressure.

The oily product obtained was isolated by column chromatography (Wako Gel C-200, eluted with toluene : ethyl acetate = 5 : 1) to give 1.2 g of the desired product as colorless oily liquid.
$n_D^{26.2}$ 1.5645

Among the pyridazinone derivatives of the present invention shown in Table 1 below, preferred are Compounds. Nos. 6, 8, 9, 12, 74, 79 and 80 wherein $R^3$ is an alkyl group and Compounds Nos. 17, 18 to 23, 26 to 33, 36, 37, 39, 40, 46 to 54, 56 to 58, 62 to 72, 75, 76, 89, 93, 98 to 101, 113, 114, 125 to 129 and 133 wherein $R^3$ is other than the alkyl group. Of these compounds, particularly preferred are Compounds Nos. 18, 31, 128 and 129.

Table 1

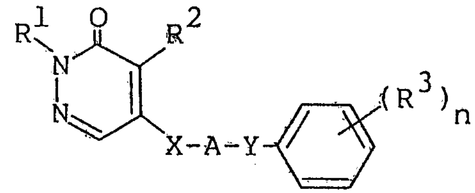

(I)

| Compound No. | $R^1$ | $R^2$ | $(R^3)_n$ 2-pos. | $(R^3)_n$ 3-pos. | $(R^3)_n$ 4-position | $(R^3)_n$ 5-pos. | $(R^3)_n$ 6-pos. | X | A | Y | Physical property |
|---|---|---|---|---|---|---|---|---|---|---|---|
| 1 | $CH_3$ | Cl | $CH_3$ | H | $n-C_5H_{11}$ | H | H | S | $-(CH_2)_2-$ | O | mp. 99-101°C |
| 2 | $CH_3$ | Cl | $CH_3$ | H | $-CH_2CH_2OC_2H_5$ | H | H | S | $-(CH_2)_2-$ | O | mp.101-103°C |
| 3 | $t-C_4H_9$ | Cl | $CH_3$ | H | Cl | H | H | S | $-(CH_2)_2-$ | O | mp. 93- 95°C |
| 4 | $t-C_4H_9$ | Cl | $CH_3$ | H | $-CH_3$ | H | H | S | $-(CH_2)_2-$ | O | mp. 99-100°C |
| 5 | $t-C_4H_9$ | Cl | $CH_3$ | H | $-C_2H_5$ | H | $CH_3$ | S | $-(CH_2)_2-$ | O | mp. 82- 84°C |
| 6 | $t-C_4H_9$ | Cl | $CH_3$ | H | $n-C_3H_7$ | H | H | S | $-(CH_2)_2-$ | O | $n_D^{16.0}$ 1.5662 |
| 7 | $t-C_4H_9$ | Cl | $CH_3$ | H | $n-C_3H_7$ | H | H | S | $-(CH_2)_4-$ | O | $n_D^{29.0}$ 1.5484 |
| 8 | $t-C_4H_9$ | Cl | $CH_3$ | H | $n-C_3H_7$ | H | Cl | S | $-(CH_2)_2-$ | O | $n_D^{25.4}$ 1.5740 |
| 9 | $t-C_4H_9$ | Cl | $CH_3$ | H | $n-C_4H_9$ | H | H | S | $-(CH_2)_2-$ | O | $n_D^{13.0}$ 1.5748 |
| 10 | $t-C_4H_9$ | Cl | $CH_3$ | H | $n-C_4H_9$ | H | H | S | $-(CH_2)_3-$ | O | $n_D^{16.0}$ 1.5616 |
| 11 | $t-C_4H_9$ | Cl | $CH_3$ | H | $i-C_4H_9$ | H | H | S | $-(CH_2)_2-$ | O | $n_D^{25.4}$ 1.5574 |

Table 1 (Contd)

| Compound No. | $R^1$ | $R^2$ | $(R^3)_n$ 2-pos. | 3-pos. | 4-position | 5-pos. | 6-pos. | X | A | Y | Physical property |
|---|---|---|---|---|---|---|---|---|---|---|---|
| 12 | $t-C_4H_9$ | Cl | $CH_3$ | H | $n-C_5H_{11}$ | H | H | S | $-(CH_2)_2-$ | O | $n_D^{29.0}$ 1.5463 |
| 13 | | | | | $n-C_8H_{17}$ | | | | | | $n_D^{13.0}$ 1.5748 |
| 14 | | | | | H | $i-C_3H_7$ | | | | | $n_D^{25.4}$ 1.5640 |
| 15 | | | | | $-CH_2CH=CH_2$ | H | | | | | $n_D^{17.6}$ 1.5870 |
| 16 | | | | | $-CH_2-C(CH_3)=CH_2$ | | | | | | $n_D^{25.4}$ 1.5667 |
| 17 | | | | | $-CH_2CH_2OCH_3$ | | | | | | $n_D^{18.2}$ 1.5765 |
| 18 | | | | | $-CH_2CH_2OC_2H_5$ | | | | | | $n_D^{16.4}$ 1.5693 |
| 19 | | | | | $-CH_2CH_2O-n-C_3H_7$ | | | | | | $n_D^{19.7}$ 1.5697 |
| 20 | | | | | $-CH_2CH_2O-i-C_3H_7$ | | | | | | $n_D^{13.0}$ 1.5687 |
| 21 | | | | | $-CH_2CH_2O-n-C_4H_9$ | | | | | | $n_D^{19.7}$ 1.5622 |
| 22 | | | | | $-CH_2CH_2OCH_2CH=CH_2$ | | | | | | $n_D^{19.7}$ 1.5762 |
| 23 | | | | | $-CH_2CH_2OCH_2C{\equiv}CH$ | | | | | | $n_D^{20.0}$ 1.5803 |
| 24 | | | | | $-CH_2CH_2OCH_2-$ 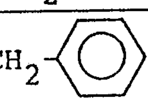 | | | | | | $n_D^{19.7}$ 1.5918 |

Table 1 (Contd)

| Compound No. | $R^1$ | $R^2$ | $(R^3)_n$ 2-pos. | $(R^3)_n$ 3-pos. | $(R^3)_n$ 4-position | $(R^3)_n$ 5-pos. | $(R^3)_n$ 6-pos. | X | A | Y | Physical property |
|---|---|---|---|---|---|---|---|---|---|---|---|
| 25 | $t-C_4H_9$ | Cl | $CH_3$ | H | $-CH=CH_2$ | H | H | S | $-(CH_2)_2-$ | O | $n_D^{29.4}$ 1.6010 |
| 26 | $t-C_4H_9$ | Cl | H | H | $-CH_2CH_2OCH_3$ | H | H | S | $-(CH_2)_2-$ | O | mp. 66- 68°C |
| 27 | $t-C_4H_9$ | Cl | H | H | $-CH_2CH_2OC_2H_5$ | H | H | S | $-(CH_2)_2-$ | O | mp. 36- 38°C |
| 28 | $t-C_4H_9$ | Br | $CH_3$ | H | $-CH_2CH_2OC_2H_5$ | H | H | S | $-(CH_2)_2-$ | O | $n_D^{14.8}$ 1.5436 |
| 29 | $t-C_4H_9$ | Br | $CH_3$ | $CH_3$ | $-CH_2CH_2OC_2H_5$ | H | H | S | $-(CH_2)_2-$ | O | mp. 78- 79°C |
| 30 | $t-C_4H_9$ | Cl | $CH_3$ | H | $-CH_2OC_2H_5$ | H | H | S | $-(CH_2)_2-$ | O | $n_D^{24.2}$ 1.5699 |
| 31 | $t-C_4H_9$ | Cl | $CH_3$ | H | $-CH_2OC_2H_5$ | H | $CH_3$ | S | $-(CH_2)_2-$ | O | $n_D^{25.2}$ 1.5681 |
| 32 | $t-C_4H_9$ | Cl | $CH_3$ | H | $-CH_2O-n-C_4H_9$ | H | $CH_3$ | S | $-(CH_2)_2-$ | O | $n_D^{25.8}$ 1.5738 |
| 33 | $t-C_4H_9$ | Cl | $CH_3$ | $CH_3$ | $-CH_2CH_2OC_2H_5$ | H | H | S | $-(CH_2)_2-$ | O | mp. 76- 79°C |
| 34 | $i-C_3H_7$ | Cl | $CH_3$ | H | $-CH_2CH_2O-C_2H_5$ | H | H | S | $-(CH_2)_2-$ | O | mp. 62- 64°C |
| 35 | $i-C_3H_7$ | Cl | $CH_3$ | H | $n-C_5H_{11}$ | H | H | S | $-(CH_2)_2-$ | O | mp. 69- 70°C |
| 36 | $t-C_4H_9$ | Cl | H | H | $-CH_2CH_2O-n-C_3H_7$ | H | H | S | $-(CH_2)_2-$ | O | mp. 42- 46°C |
| 37 | $t-C_4H_9$ | Cl | H | H | $-CH_2CH_2O-n-C_4H_9$ | H | H | S | $-(CH_2)_2-$ | O | mp. 46- 48°C |
| 38 | $t-C_4H_9$ | Cl | H | H | $n-C_3H_7$ | H | H | S | $-(CH_2)_2-$ | O | $n_D^{23.8}$ 1.5758 |
| 39 | $t-C_4H_9$ | Cl | H | H | $-(CH_2)_2OCH_3$ | H | H | O | $-(CH_2)_2-$ | O | mp. 77- 81°C |

Table 1 (Contd)

| Compound No. | $R^1$ | $R^2$ | $(R^3)_n$ 2-pos. | 3-pos. | 4-position | 5-pos. | 6-pos. | X | A | Y | Physical property |
|---|---|---|---|---|---|---|---|---|---|---|---|
| 40 | $t-C_4H_9$ | Cl | H | H | $-(CH_2)_2O-n-C_3H_7$ | H | H | O | $-(CH_2)_2-$ | O | mp. 71- 73°C |
| 41 | $t-C_4H_9$ | Cl | H | H | $-(CH_2)_2-SC_2H_5$ | H | H | S | $-(CH_2)_2-$ | O | $n_D^{25.2}$ 1.5866 |
| 42 | $t-C_4H_9$ | Cl | H | H | $-(CH_2)_2-SC_2H_5$ | H | H | O | $-(CH_2)_2-$ | O | $n_D^{25.2}$ 1.5737 |
| 43 | $t-C_4H_9$ | Cl | $CH_3$ | H | $-CH=CH_2$ | H | H | O | $-(CH_2)_2-$ | O | mp. 93- 95°C |
| 44 | $t-C_4H_9$ | Cl | $CH_3$ | H | $n-C_4H_9$ | H | H | O | $-CH(C_2H_5)-CH_2-$ | O | $n_D^{22.8}$ 1.5390 |
| 45 | $t-C_4H_9$ | Cl | $CH_3$ | H | $n-C_5H_{11}$ | H | H | O | $-(CH_2)_2-$ | O | mp. 46- 51°C |
| 46 | $t-C_4H_9$ | Cl | $CH_3$ | H | $-(CH_2)_2-OCH_3$ | H | H | O | $-(CH_2)_2-$ | O | $n_D^{26.0}$ 1.5496 |
| 47 | $t-C_4H_9$ | Cl | $CH_3$ | H | $-(CH_2)_2-OC_2H_5$ | H | H | O | $-(CH_2)_2-$ | O | $n_D^{24.0}$ 1.5438 |
| 48 | $t-C_4H_9$ | Br | $CH_3$ | H | $-(CH_2)_2-OC_2H_5$ | H | H | O | $-(CH_2)_2-$ | O | mp. 45- 46°C |
| 49 | $t-C_4H_9$ | Cl | $CH_3$ | H | $-(CH_2)_2O-n-C_3H_7$ | H | H | O | $-(CH_2)_2-$ | O | $n_D^{22.2}$ 1.5410 |
| 50 | $t-C_4H_9$ | Cl | $CH_3$ | H | $-(CH_2)_2O-n-C_4H_9$ | H | H | O | $-(CH_2)_2-$ | O | $n_D^{20.4}$ 1.5389 |
| 51 | $t-C_4H_9$ | Cl | $CH_3$ | H | $-(CH_2)_2OCH_2CH=CH_2$ | H | H | O | $-(CH_2)_2-$ | O | $n_D^{22.0}$ 1.5508 |
| 52 | $t-C_4H_9$ | Cl | $CH_3$ | H | $-(CH_2)_2OCH_2C\equiv CH$ | H | H | O | $-(CH_2)_2-$ | O | $n_D^{22.2}$ 1.5570 |

Table 1 (Contd)

| Compound No. | $R^1$ | $R^2$ | $(R^3)_n$ 2-pos. | 3-pos. | 4-position | 5-pos. | 6-pos. | X | A | Y | Physical property |
|---|---|---|---|---|---|---|---|---|---|---|---|
| 53 | $t-C_4H_9$ | Cl | $CH_3$ | H | $-(CH_2)_2O(CH_2)_2-OC_2H_5$ | H | H | O | $-(CH_2)_2-$ | O | $n_D^{26.0}$ 1.5351 |
| 54 | $t-C_4H_9$ | Cl | $CH_3$ | H | $-(CH_2)_2OCH(CH_3)OC_2H_5$ | H | H | O | $-(CH_2)_2-$ | O | $n_D^{26.1}$ 1.5341 |
| 55 | $t-C_4H_9$ | Cl | $CH_3$ | H | $n-C_3H_7$ | H | H | S | $-(CH_2)_2-$ | S | $n_D^{26.8}$ 1.5910 |
| 56 | $t-C_4H_9$ | Cl | $CH_3$ | H | $-(CH_2)_4OCH_3$ | H | H | O | $-(CH_2)_2-$ | O | $n_D^{26.4}$ 1.5420 |
| 57 | $t-C_4H_9$ | Cl | $CH_3$ | H | $-(CH_2)_4OCH_3$ | H | H | S | $-(CH_2)_2-$ | O | $n_D^{26.4}$ 1.5644 |
| 58 | $t-C_4H_9$ | Cl | $CH_3$ | H | $-(CH_2)_2O(CH_2)_2-OC_2H_5$ | H | H | S | $-(CH_2)_2-$ | O | $n_D^{26.5}$ 1.5590 |
| 59 | $t-C_4H_9$ | Cl | $CH_3$ | H | $-CH_2CH_2F$ | H | H | S | $-(CH_2)_2-$ | O | mp. 79- 82°C |
| 60 | $t-C_4H_9$ | Cl | Cl | H | $-CH_3$ | H | H | S | $-(CH_2)_2-$ | O | mp.102-104°C |
| 61 | $t-C_4H_9$ | Cl | Cl | H | $n-C_3H_7$ | H | H | S | $-(CH_2)_2-$ | O | $n_D^{23.6}$ 1.5829 |
| 62 | $t-C_4H_9$ | Cl | Cl | H | $-(CH_2)_2-OCH_3$ | H | H | O | $-(CH_2)_2-$ | O | $n_D^{25.5}$ 1.5596 |
| 63 | $t-C_4H_9$ | Cl | Cl | H | $-(CH_2)_2-OCH_3$ | H | H | S | $-(CH_2)_2-$ | O | $n_D^{25.0}$ 1.5714 |
| 64 | $t-C_4H_9$ | Cl | Cl | H | $-(CH_2)_2-OCH_3$ | $CH_3$ | H | O | $-(CH_2)_2-$ | O | mp. 83- 85°C |

Table 1 (Contd)

| Compound No. | $R^1$ | $R^2$ | $(R^3)_n$ 2-pos. | 3-pos. | 4-position | 5-pos. | 6-pos. | X | A | Y | Physical property |
|---|---|---|---|---|---|---|---|---|---|---|---|
| 65 | $t-C_4H_9$ | Cl | Cl | H | $-(CH_2)_2OC_2H_5$ | $CH_3$ | H | O | $-(CH_2)_2-$ | O | mp. 79- 81°C |
| 66 | $t-C_4H_9$ | Cl | $CH_3$ | H | $-(CH_2)_2OCH_3$ | $CH_3$ | H | S | $-(CH_2)_2-$ | O | $n_D^{27.4}$ 1.5743 |
| 67 | $t-C_4H_9$ | Cl | $CH_3$ | H | $-(CH_2)_2OC_2H_5$ | $CH_3$ | H | S | $-(CH_2)_2-$ | O | $n_D^{19.4}$ 1.5700 |
| 68 | $t-C_4H_9$ | Cl | $CH_3$ | H | $-CH_2OC_2H_5$ | H | $CH_3$ | O | $-(CH_2)_2-$ | O | mp.102-105°C |
| 69 | $t-C_4H_9$ | Cl | $CH_3$ | H | $-(CH_2)_2OCH_3$ | H | $CH_3$ | O | $-(CH_2)_2-$ | O | mp. 89- 91°C |
| 70 | $t-C_4H_9$ | Cl | $CH_3$ | H | $-(CH_2)_2OC_2H_5$ | H | $CH_3$ | O | $-(CH_2)_2-$ | O | mp. 88- 90°C |
| 71 | $t-C_4H_9$ | Cl | $CH_3$ | H | $-(CH_2)_2OCH_3$ | H | $CH_3$ | S | $-(CH_2)_2-$ | O | $n_D^{20.2}$ 1.5708 |
| 72 | $t-C_4H_9$ | Cl | $CH_3$ | H | $-(CH_2)_2OC_2H_5$ | H | $CH_3$ | S | $-(CH_2)_2-$ | O | $n_D^{20.2}$ 1.5647 |
| 73 | $t-C_4H_9$ | Cl | Cl | H | $-CH_3$ | H | Cl | S | $-(CH_2)_2-$ | O | mp. 98-100°C |
| 74 | $t-C_4H_9$ | Cl | Cl | H | $n-C_3H_7$ | H | Cl | S | $-(CH_2)_2-$ | O | $n_D^{23.6}$ 1.5822 |
| 75 | $t-C_4H_9$ | Cl | Cl | H | $-(CH_2)_2OCH_3$ | H | Cl | O | $-(CH_2)_2-$ | O | $n_D^{25.4}$ 1.5607 |
| 76 | $t-C_4H_9$ | Cl | Cl | H | $-(CH_2)_2OCH_3$ | H | Cl | S | $-(CH_2)_2-$ | O | $n_D^{25.0}$ 1.5861 |
| 77 | $t-C_4H_9$ | Cl | Cl | H | Cl | H | Cl | S | $-(CH_2)_2-$ | O | mp.111-112°C |
| 78 | $t-C_4H_9$ | Cl | $CH_3$ | H | $-C_2H_5$ | H | Cl | S | $-(CH_2)_2-$ | O | mp. 69- 73°C |
| 79 | $t-C_4H_9$ | Cl | $CH_3$ | H | $n-C_4H_9$ | H | Cl | S | $-(CH_2)_2-$ | O | $n_D^{23.6}$ 1.5710 |

Table 1 (Contd)

| Compound No. | $R^1$ | $R^2$ | $(R^3)_n$ 2-pos. | 3-pos. | 4-position | 5-pos. | 6-pos. | X | A | Y | Physical property |
|---|---|---|---|---|---|---|---|---|---|---|---|
| 80 | $t\text{-}C_4H_9$ | Cl | $CH_3$ | H | $n\text{-}C_5H_{11}$ | H | Cl | S | $-(CH_2)_2-$ | O | $n_D^{23.6}$ 1.5681 |
| 81 | | | Br | | $-(CH_2)_2OCH_3$ | | Br | | | | $n_D^{25.0}$ 1.5862 |
| 82 | | | | | | | | O | | | $n_D^{25.0}$ 1.5686 |
| 83 | | | $C_2H_5$ | | $n\text{-}C_4H_9$ | | Cl | S | | | $n_D^{25.0}$ 1.5710 |
| 84 | | | | | | | | O | | | mp. 78- 80°C |
| 85 | | | $CH_3$ | | $-CH_2CF_2CH_3$ | | H | | | | $n_D^{28.6}$ 1.5342 |
| 86 | | | | | $-CH(CH_2)_3CH_3$ with F on CH | | Cl | S | | | $n_D^{25.4}$ 1.5627 |
| 87 | | | | | $-CH_2CHOCH_2-$ 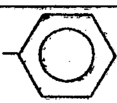 with $CH_3$ on CH | | H | O | | | $n_D^{28.6}$ 1.5575 |
| 88 | | | | | $-CH(CH_2)_3CH_3$ with Cl on CH | | Cl | S | | | $n_D^{25.4}$ 1.5733 |
| 89 | | | | $CH_3$ | $-(CH_2)_2OC_2H_5$ | | | O | | | mp. 80- 83°C |
| 90 | | | Cl | H | $-CH_2SCH_3$ | | | S | | | $n_D^{26.0}$ 1.5994 |
| 91 | | | | | $-CH_2SC_2H_5$ | | | | | | $n_D^{26.2}$ 1.5924 |

Table 1 (Contd)

| Compound No. | $R^1$ | $R^2$ | $(R^3)_n$ 2-pos. | 3-pos. | 4-position | 5-pos. | 6-pos. | X | A | Y | Physical property |
|---|---|---|---|---|---|---|---|---|---|---|---|
| 92 | $t-C_4H_9$ | Cl | F | F | F | F | F | S | $-(CH_2)_2-$ | O | mp.100-102°C |
| 93 | | | $CH_3$ | H | $-CH_2CH_2OC_2H_5$ | H | Cl | | | | $n_D^{25.6}$ 1.5694 |
| 94 | | | | | $n-C_3H_7$ | | H | O | $-\underset{\vert}{C}HCH_2-$ $CH_3$ | | mp. 80- 82°C |
| 95 | | | | | $n-C_4H_9$ | | Cl | | | | $n_D^{25.8}$ 1.5443 |
| 96 | | | | | $n-C_5H_{11}$ | | | | $-(CH_2)_2-$ | | mp. 66- 69°C |
| 97 | | | | | $-CH_2Cl$ | | | S | | | mp. 90- 93°C |
| 98 | | | Cl | | $-CH_2OCH_3$ | | | | | | $n_D^{23.1}$ 1.5924 |
| 99 | | | | | $-CH_2OC_2H_5$ | | | | | | $n_D^{25.8}$ 1.5861 |
| 100 | | | | | $-CH_2O-n-C_3H_7$ | | | | | | $n_D^{23.0}$ 1.5751 |
| 101 | | | | | $-CH_2O-i-C_3H_7$ | | | | | | $n_D^{23.0}$ 1.5721 |
| 102 | | | | | $-CH_2SO_2C_2H_5$ | | | | | | $n_D^{24.9}$ 1.5789 |
| 103 | | | $CH_3$ | | $-CH_2NH-t-C_4H_9 \cdot HCl$ | | $CH_3$ | | | | $n_D^{24.4}$ 1.5612 |
| 104 | | | Cl | | 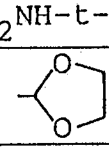 | | Cl | | | | $n_D^{26.2}$ 1.5915 |

Table 1 (Contd)

| Compound No. | $R^1$ | $R^2$ | $(R^3)_n$ 2-pos. | 3-pos. | 4-position | 5-pos. | 6-pos. | X | A | Y | Physical property |
|---|---|---|---|---|---|---|---|---|---|---|---|
| 105 | $t-C_4H_9$ | Cl | Cl | H | 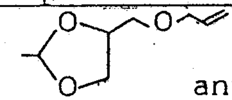 anti | H | Cl | S | $-(CH_2)_2-$ | O | $n_D^{25.4}$ 1.5673 |
| 106 | $t-C_4H_9$ | Cl | Cl | H | 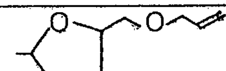 syn | H | Cl | S | $-(CH_2)_2-$ | O | $n_D^{25.6}$ 1.5638 |
| 107 | $t-C_4H_9$ | Cl | $CH_3$ | H | 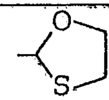 | H | $CH_3$ | S | $-(CH_2)_2-$ | O | mp.148-153°C |
| 108 | $t-C_4H_9$ | Cl | Cl | H | $-CH=NOCH_3$ | H | Cl | S | $-(CH_2)_2-$ | O | mp.132-136°C |
| 109 | $t-C_4H_9$ | Cl | Cl | H | -CHO | H | Cl | S | $-(CH_2)_2-$ | O | mp.117-119°C |
| 110 | $t-C_4H_9$ | Cl | Cl | H | $-CH_2OCH_2CF_3$ | H | Cl | S | $-(CH_2)_2-$ | O | $n_D^{18.6}$ 1.5592 |
| 111 | $t-C_4H_9$ | Cl | Cl | H | $-CH_2O-$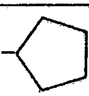 | H | Cl | S | $-(CH_2)_2-$ | O | $n_D^{25.3}$ 1.5750 |
| 112 | $t-C_4H_9$ | Cl | Cl | H | $-CH_2OCH_2CH_2SC_2H_5$ | H | Cl | S | $-(CH_2)_2-$ | O | mp. 73- 77°C |
| 113 | $t-C_4H_9$ | Cl | Cl | H | $-CH_2OC_2H_5$ | H | $CH_3$ | S | $-(CH_2)_2-$ | O | $n_D^{25.2}$ 1.5724 |
| 114 | $t-C_4H_9$ | Cl | Cl | H | $-CH_2-$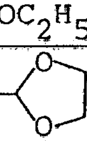 | H | $CH_3$ | S | $-(CH_2)_2-$ | O | mp. 82- 83°C |
| 115 | $t-C_4H_9$ | Cl | Cl | H | 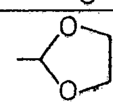 | H | $CH_3$ | S | $-(CH_2)_2-$ | O | mp.133-134°C |

Table 1 (Contd)

| Compound No. | $R^1$ | $R^2$ | $(R^3)_n$ 2-pos. | 3-pos. | 4-position | 5-pos. | 6-pos. | X | A | Y | Physical property |
|---|---|---|---|---|---|---|---|---|---|---|---|
| 116 | | | | | $-CH_2CH_2OH$ | | | | | | mp.180-182°C |
| 117 | | | | | $-CH_2CH_2O\overset{O}{\overset{\|}{C}}CH_3$ | | | | | | $n_D^{26.2}$ 1.5645 |
| 118 | | | | | $-CH_2CH_2O\overset{O}{\overset{\|}{C}}-t-C_4H_9$ | | | | | | $n_D^{27.0}$ 1.5538 |
| 119 | | | | | $-CH_2CH_2O\overset{O}{\overset{\|}{C}}-$ 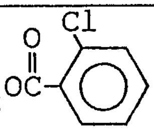 | | | | | | $n_D^{26.2}$ 1.5751 |
| 120 | | | | | $-CH_2COOC_2H_5$ | | | | | | $n_D^{24.4}$ 1.5616 |
| 121 | $t-C_4H_9$ | Cl | Cl | H | $-\overset{O}{\overset{\|}{C}}-n-C_4H_9$ | H | $CH_3$ | S | $-(CH_2)_2-$ | O | $n_D^{25.2}$ 1.5757 |
| 122 | | | | | $-\overset{OH}{\overset{\|}{C}}H-n-C_4H_9$ | | | | | | $n_D^{25.4}$ 1.5614 |
| 123 | | | | | $-C(=NOCH_3)-n-C_4H_9$ | | | | | | $n_D^{27.2}$ 1.5700 |
| 124 | | | | | $n-C_4H_9$ | | | O | $-CH_2\underset{CH_3}{\underset{\|}{C}}H-$ | | $n_D^{22.8}$ 1.5479 |
| 125 | | | $CH_3$ | | $-\underset{CH_3}{\underset{\|}{C}}HOCH_3$ | | | S | $-(CH_2)_2-$ | | $n_D^{17.4}$ 1.5603 |

Table 1 (Contd)

| Compound No. | $R^1$ | $R^2$ | $(R^3)_n$ | | | | | X | A | Y | Physical property |
|---|---|---|---|---|---|---|---|---|---|---|---|
| | | | 2-pos. | 3-pos. | 4-position | 5-pos. | 6-pos. | | | | |
| 126 | $t-C_4H_9$ | Cl | $CH_3$ | H | $-CH_2OCH_2C{\equiv}CH$ | H | $CH_3$ | S | $-(CH_2)_2-$ | O | $n_D^{15.8}$ 1.5692 |
| 127 | | | | | $-CH_2-O-$ 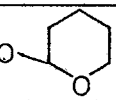 | | | | | | mp. 90- 92°C |
| 128 | | | | | $-CH_2CHOCH_3$<br>$CH_3$ | | | | | | $n_D^{19.6}$ 1.5612 |
| 129 | | | | | $-CH_2CHOCH_3$<br>$C_2H_5$ | | | | | | $n_D^{19.2}$ 1.5567 |
| 130 | | | | | 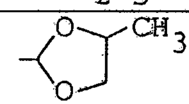 | | | | | | mp. 95- 99°C |
| 131 | | | | | 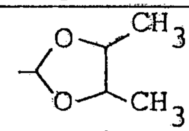 | | | | | | mp.109-111°C |
| 132 | | | | | 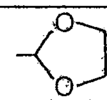 | | | | | | mp.119-120°C |
| 133 | | | | | $-CH_2-$ 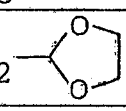 | | | | | | $n_D^{26.0}$ 1.5714 |
| 134 | | | | | 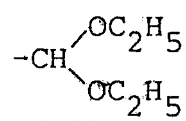 | | | | | | mp.104-106°C |

Table 1 (Contd)

| Compound No. | $R^1$ | $R^2$ | $(R^3)_n$ 2-pos. | 3-pos. | 4-position | 5-pos. | 6-pos. | X | A | Y | Physical property |
|---|---|---|---|---|---|---|---|---|---|---|---|
| 135 | $t-C_4H_9$ | Cl | $NO_2$ | H | 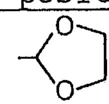 | H | $OCH_3$ | S | $-(CH_2)_2-$ | O | $n_D^{20.2}$ 1.5781 |
| 136 | $t-C_4H_9$ | Cl | $CH_3$ | H | $-CH_2CH=NOC_2H_5$ | H | $CH_3$ | S | $-(CH_2)_2-$ | O | |
| 137 | $t-C_4H_9$ | Cl | Cl | H | $-CH_2CH(C_2H_5)OCH_3$ | H | $CH_3$ | S | $-(CH_2)_2-$ | O | |
| 138 | $t-C_4H_9$ | Cl | Cl | H | $-CH_2CH(C_2H_5)OCH_3$ | H | Cl | S | $-(CH_2)_2-$ | O | |

Example 16

5 parts by weight of the compound of the Compound No. 1, 35 parts by weight of bentonite, 57 parts by weight of talc, 1 part by weight of Neopelex Powder (trade name, produced by Kao-Atlas Co.) and 2 parts by weight of sodium lignosulfate were homogeneously mixed, then kneaded with addition of a small amount of water, followed by granulation and drying to obtain granules.

Example 17

50 parts by weight of the compound of the Compound No. 17, 48 parts by weight of kaolin and 2 parts by weight of Neopelex Powder (trade name, produced by Kao-Atlas Co.) were homogeneously mixed, followed by pulverization, to obtain wettable powder.

Example 18

To 20 parts by weight of the compound of the Compound No. 22 and 70 parts by weight of xylene were added 10 parts by weight of Toxanon (trade name, produced by Sanyo Kasei Kogyo, Co.), and homogeneously mixed and dissolved to obtain an emulsion.

Example 19

5 parts by weight of the compound of the Compound No. 12, 50 parts by weight of talc and 45 parts by weight of kaolin were homogeneously mixed to obtain powder.

Test 1 Activity test against diamondback moth (organic phosphorus agent resistance)

In plastic cups of 10 cm in diameter, cabbage leaf strips (5 cm x 5 cm) were placed, while the medicinal solutions prepared by forming the compounds shown in Table 1 into preparations similarly as in Example 17 and diluting with water containing a surfactant (0.01 %) to 300 ppm were sprayed each in 5 ml in a spraying tower. After drying on air, 10 third-instar larvae of diamondback moth were provided for test, left to stand in a thermostat chamber at 25 °C. After 2 days, the numbers of alive and dead insects were examined to determine the insecticide ratio. The results are shown in Table 2.

In the evaluation of insecticide ratio, those with insecticide ratio of 100 % are shown as 5, 99 to 80 % as 4, 79 to 60 % as 3, 59 to 40 % as 2, 39 to 20 % as 1 and 19 to 0 as 0, and these also apply to the following tests.

Further, the following compound described in Japanese Provisional Patent Publication No. 130275/1986 was prepared and tested similarly to the above procedure, as reference A:

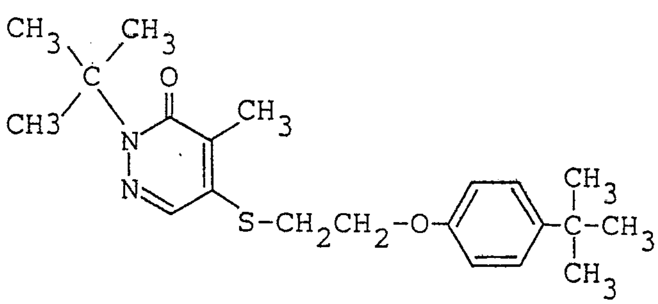

[A]

Table 2

| Compound No. | Effect | Compound No. | Effect | Compound No. | Effect |
|---|---|---|---|---|---|
| 5 | 5 | 48 | 5 | 78 | 5 |
| 6 | 5 | 49 | 5 | 79 | 5 |
| 8 | 5 | 50 | 5 | 80 | 5 |
| 9 | 5 | 51 | 5 | 81 | 5 |
| 11 | 5 | 52 | 5 | 82 | 4 |
| 12 | 5 | 53 | 5 | 83 | 5 |
| 13 | 4 | 54 | 4 | 85 | 5 |
| 14 | 5 | 56 | 5 | 86 | 5 |
| 15 | 5 | 57 | 5 | 87 | 5 |
| 16 | 5 | 58 | 5 | 88 | 5 |
| 17 | 5 | 59 | 5 | 90 | 5 |
| 18 | 5 | 61 | 5 | 91 | 5 |
| 19 | 4 | 62 | 5 | 93 | 5 |
| 20 | 4 | 63 | 5 | 98 | 5 |
| 22 | 5 | 64 | 5 | 99 | 5 |
| 23 | 5 | 65 | 5 | 100 | 5 |
| 26 | 4 | 66 | 5 | 101 | 5 |
| 27 | 4 | 67 | 5 | 102 | 5 |
| 28 | 5 | 71 | 5 | 103 | 5 |
| 29 | 5 | 72 | 5 | 104 | 5 |
| 38 | 4 | 74 | 5 | 105 | 5 |
| 45 | 5 | 75 | 5 | 106 | 5 |
| 46 | 5 | 76 | 5 | 110 | 4 |
| 47 | 5 | 77 | 4 | 113 | 5 |

Table 2 (Contd)

| Compound No. | Effect | Compound No. | Effect | Compound No. | Effect |
|---|---|---|---|---|---|
| 114 | 5 | 126 | 5 | 132 | 5 |
| 115 | 5 | 127 | 5 | 133 | 5 |
| 118 | 4 | 128 | 5 | | |
| 120 | 5 | 129 | 5 | | |
| 121 | 5 | 130 | 5 | | |
| 124 | 5 | 131 | 5 | Reference A | 1 |

Test 2 Activity test against brown planthopper

In the medicinal solutions prepared by forming the compounds shown in Table 1 into preparations similarly as in Example 17 and diluting with water containing a surfactant (0.01 %) to 300 ppm, rice seedlings were dipped for 30 seconds and after drying on air inserted into a glass cylinder. Ten third-instar larvae of brown planthopper were freed, and left to stand in a thermostat chamber at 25 °C with attachment of a porous stopper. After 2 days, the numbers of alive and dead insects were examined to determine the insecticide ratio The results are shown in Table 3.

Further, the compound represented by the foregoing formula [A] which is described in Japanese Provisional Patent Publication No 130275/1986 was prepared and tested similarly to the above procedure, as reference B.

Table 3

| Compound No. | Effect | Compound No. | Effect | Compound No. | Effect |
|---|---|---|---|---|---|
| 5 | 4 | 31 | 5 | 81 | 4 |
| 6 | 5 | 38 | 4 | 85 | 4 |
| 8 | 5 | 45 | 4 | 93 | 4 |
| 9 | 5 | 46 | 4 | 98 | 5 |
| 11 | 5 | 56 | 4 | 101 | 4 |
| 12 | 4 | 57 | 4 | 104 | 4 |
| 14 | 4 | 61 | 5 | 113 | 4 |
| 15 | 5 | 63 | 5 | 114 | 4 |
| 16 | 5 | 71 | 5 | 117 | 4 |
| 17 | 5 | 72 | 4 | 125 | 5 |
| 18 | 4 | 74 | 5 | 128 | 4 |
| 20 | 4 | 76 | 5 | 129 | 4 |
| 22 | 4 | 78 | 5 | 133 | 4 |
| 26 | 4 | 79 | 5 | | |
| 28 | 4 | 80 | 5 | Reference B | 0 |

Test 3 Activity test against green rice leafhopper (organic phosphorus agent resistance)

In the medicinal solutions prepared by forming the compounds shown in Table 1 into preparations similarly as in Examples 17 and 18 and diluting with water containing a surfactant (0.01 %) to 300 ppm, rice seedlings were dipped for 30 seconds and after drying on air inserted into a glass cylinder. Ten four-instar larvae of green rice leafhopper were freed, and left to stand in a thermostat chamber at 25°C with attachment of a porous stopper. After 2 days, the numbers of alive and dead insects were examined to determine the insecticide ratio. The results are shown in Table 4 and Table 4'.

Further, the compounds represented by the foregoing formula [A] which are described in Japanese Provisional Patent Publication No. 130275/1986 were prepared and tested similarly to the above precedures, as reference C and D.

Table 4

(preparation method: Example 18)

| Compound No. | Effect | Compound No. | Effect | Compound No. | Effect |
|---|---|---|---|---|---|
| 41 | 4 | 61 | 5 | 78 | 5 |
| 42 | 5 | 62 | 5 | 79 | 5 |
| 45 | 5 | 63 | 5 | 80 | 5 |
| 46 | 5 | 64 | 4 | 81 | 5 |
| 47 | 5 | 65 | 4 | 82 | 5 |
| 48 | 5 | 66 | 4 | 83 | 5 |
| 49 | 4 | 67 | 5 | 84 | 4 |
| 50 | 4 | 68 | 5 | 85 | 5 |
| 51 | 4 | 69 | 5 | 86 | 5 |
| 52 | 4 | 70 | 5 | 87 | 5 |
| 53 | 5 | 71 | 5 | 88 | 5 |
| 56 | 5 | 72 | 5 | 90 | 5 |
| 57 | 5 | 73 | 4 | 91 | 5 |
| 58 | 5 | 74 | 5 | 93 | 5 |
| 59 | 5 | 75 | 5 | 94 | 4 |
| 60 | 4 | 76 | 5 | 95 | 4 |

Table 4 (contd)

(preparation method: Example 18)

| Compound No. | Effect | Compound No. | Effect | Compound No. | Effect |
|---|---|---|---|---|---|
| 98 | 5 | 112 | 4 | 127 | 4 |
| 99 | 5 | 113 | 5 | 128 | 5 |
| 100 | 5 | 114 | 5 | 129 | 5 |
| 101 | 5 | 120 | 4 | 130 | 5 |
| 102 | 5 | 121 | 5 | 131 | 5 |
| 103 | 4 | 122 | 5 | 132 | 5 |
| 104 | 5 | 123 | 5 | 133 | 5 |
| 105 | 5 | 124 | 5 | | |
| 107 | 5 | 125 | 5 | | |
| 108 | 4 | 126 | 5 | Reference C | 0 |

Table 4'

(preparation method: Example 17)

| Compound No. | Effect | Compound No. | Effect | Compound No. | Effect |
|---|---|---|---|---|---|
| 3 | 4 | 11 | 5 | 18 | 4 |
| 5 | 5 | 14 | 5 | 28 | 4 |
| 6 | 4 | 15 | 4 | 31 | 5 |
| 8 | 5 | 16 | 4 | | |
| 9 | 5 | 17 | 5 | Reference D | 0 |

Test 4 Activity test against adult female citrus red mite (organic phosphorus agent resistance)

On filter papers soaked with water, mulberry leaf strips of 20 mm in diameter were placed and 10 adult female citrus red mites were inoculated. On the other hand, the medicinal solutions prepared by forming the compounds shown in Table 1 into preparations similarly as in Example 17 and diluting with water containing a surfactant (0.01 %) to 300 ppm were sprayed each in 5 ml in a spraying tower. After the treatment, each of the strips was left to stand in a thermostat chamber at 25 °C, and after 3 days, the numbers of alive and dead mites were examined to determine the acaricide ratio. The results are shown in Table 5.

Further, the compound represented by the foregoing formula [A] which is described in Japanese Provisional Patent Publication No. 130275/1986 was prepared and tested similarly to the above precedure, as reference E.

Table 5

| Compound No. | Effect | Compound No. | Effect | Compound No. | Effect |
|---|---|---|---|---|---|
| 3 | 4 | 14 | 5 | 23 | 5 |
| 5 | 4 | 15 | 5 | 24 | 5 |
| 6 | 5 | 16 | 5 | 25 | 5 |
| 8 | 5 | 17 | 4 | 26 | 5 |
| 9 | 5 | 18 | 4 | 27 | 5 |
| 10 | 5 | 19 | 5 | 28 | 5 |
| 11 | 5 | 20 | 5 | 29 | 5 |
| 12 | 5 | 21 | 5 | 30 | 5 |
| 13 | 5 | 22 | 5 | 31 | 5 |

Table 5

| Compound No. | Effect | Compound No. | Effect | Compound No. | Effect |
|---|---|---|---|---|---|
| 32 | 5 | 64 | 5 | 93 | 5 |
| 33 | 5 | 65 | 5 | 94 | 4 |
| 34 | 4 | 66 | 5 | 95 | 4 |
| 35 | 4 | 67 | 5 | 96 | 5 |
| 36 | 5 | 68 | 5 | 98 | 5 |
| 37 | 5 | 69 | 5 | 99 | 5 |
| 38 | 5 | 70 | 5 | 100 | 5 |
| 41 | 5 | 71 | 5 | 101 | 5 |
| 42 | 5 | 72 | 5 | 103 | 5 |
| 45 | 5 | 74 | 5 | 104 | 5 |
| 46 | 5 | 75 | 5 | 105 | 5 |
| 47 | 5 | 76 | 5 | 108 | 5 |
| 48 | 5 | 78 | 5 | 110 | 5 |
| 49 | 5 | 79 | 5 | 111 | 5 |
| 50 | 5 | 80 | 5 | 112 | 5 |
| 51 | 5 | 81 | 5 | 113 | 5 |
| 52 | 5 | 82 | 5 | 114 | 5 |
| 53 | 5 | 83 | 5 | 115 | 4 |
| 56 | 5 | 85 | 5 | 117 | 5 |
| 57 | 5 | 86 | 5 | 118 | 4 |
| 58 | 5 | 87 | 5 | 119 | 5 |
| 61 | 5 | 88 | 5 | 120 | 4 |
| 62 | 5 | 90 | 4 | 121 | 4 |
| 63 | 5 | 91 | 4 | 122 | 5 |

Table 5 (contd)

| Compound No. | Effect | Compound No. | Effect | Compound No. | Effect |
|---|---|---|---|---|---|
| 124 | 5 | 128 | 5 | 133 | 5 |
| 125 | 5 | 129 | 5 | | |
| 126 | 5 | 130 | 5 | | |
| 127 | 5 | 131 | 4 | Reference E | 0 |

Test 5 Activity test against citrus red mite egg (organic phosphorus agent resistance)

On filter papers soaked with water, mulberry leaf strips of 20 mm in diameter were placed and 5 adult female citrus red mites were inoculated and permitted to lay eggs for one day. On the other hand, the medicinal solutions prepared by forming the compounds shown in Table 1 into preparations similarly as in Example 17 and diluting with water containing a surfactant (0.01 %) to 300 ppm were sprayed each in 5 ml in a spraying tower. After the treatment, each of the strips was left to stand in a thermostat chamber at 25 °C, and after 10 days, the numbers of non-hatched eggs were examined to determine the egg killing ratio. The egg killing ratio was evaluated as same as the insecticide ratio described in Test 1. The results are shown in Table 6.

Further, the compound represented by the foregoing formula [A] which is described in Japanese Provisional Patent Publication No. 130275/1986 was prepared and tested similarly to the above procedure, as reference F.

Table 6

| Compound No. | Effect | Compound No. | Effect | Compound No. | Effect |
|---|---|---|---|---|---|
| 1 | 5 | 29 | 5 | 62 | 5 |
| 3 | 4 | 30 | 5 | 63 | 5 |
| 5 | 5 | 31 | 5 | 64 | 5 |
| 6 | 5 | 32 | 5 | 65 | 5 |
| 8 | 5 | 33 | 5 | 66 | 5 |
| 9 | 5 | 34 | 5 | 67 | 5 |
| 10 | 5 | 35 | 4 | 68 | 5 |
| 11 | 5 | 36 | 5 | 69 | 5 |
| 12 | 5 | 37 | 5 | 70 | 5 |
| 13 | 5 | 38 | 5 | 71 | 5 |
| 14 | 5 | 41 | 5 | 72 | 5 |
| 15 | 5 | 42 | 5 | 74 | 5 |
| 16 | 5 | 45 | 5 | 75 | 5 |
| 17 | 5 | 46 | 5 | 76 | 5 |
| 18 | 5 | 47 | 5 | 78 | 5 |
| 19 | 5 | 48 | 5 | 79 | 5 |
| 20 | 5 | 49 | 5 | 80 | 5 |
| 21 | 5 | 50 | 5 | 81 | 5 |
| 22 | 5 | 51 | 5 | 83 | 5 |
| 23 | 5 | 52 | 5 | 85 | 5 |
| 24 | 5 | 53 | 5 | 86 | 4 |
| 25 | 5 | 56 | 5 | 87 | 5 |
| 26 | 5 | 57 | 5 | 90 | 5 |
| 27 | 5 | 58 | 5 | 91 | 4 |
| 28 | 5 | 61 | 5 | 93 | 5 |

Table 6 (contd)

| Compound No. | Effect | Compound No. | Effect | Compound No. | Effect |
|---|---|---|---|---|---|
| 94 | 4 | 111 | 5 | 124 | 5 |
| 95 | 4 | 112 | 5 | 125 | 5 |
| 96 | 4 | 113 | 5 | 126 | 5 |
| 98 | 5 | 114 | 5 | 127 | 5 |
| 99 | 5 | 115 | 5 | 128 | 5 |
| 100 | 5 | 117 | 5 | 129 | 5 |
| 101 | 5 | 118 | 5 | 130 | 5 |
| 104 | 5 | 119 | 5 | 131 | 4 |
| 105 | 5 | 120 | 4 | 133 | 5 |
| 108 | 5 | 121 | 4 | | |
| 110 | 5 | 122 | 4 | Reference F | 0 |

Test 6 Activity test against adult female two-spotted spider mite (organic phosphorous agent resistance)

On filter papers soaked with water, kidney bean leaf strips of 20 mm in diameter were placed and 10 adult female two-spotted spider mites were inoculated. The leaf strips were dipped for 15 seconds in medical solutions prepared by forming the compounds shown in Table 1 into preparation similarly as in Example 17 and diluting with water containing a surfactant (0.01 %) to 300 ppm. After drying on air, the leaf strips were left to stand in a thermostat chamber at 25 °C. After 3 days, the numbers of alive and dead insects were examined to determine the acaricide ratio. The acaricide ratio was evaluated as same as the insecticide ratio described in Test 1. The results are shown in Table 7.

Further, the compound represented by the foregoing formula [A] which is described in Japanese Provisional Patent Publication No. 130275/1986 was prepared and tested similarly to the above procedure, as reference G.

Table 7

| Compound No. | Effect | Compound No. | Effect | Compound No. | Effect |
|---|---|---|---|---|---|
| 3 | 4 | 27 | 5 | 53 | 5 |
| 6 | 5 | 28 | 5 | 55 | 5 |
| 8 | 5 | 29 | 5 | 56 | 5 |
| 9 | 5 | 30 | 5 | 57 | 5 |
| 10 | 5 | 31 | 5 | 58 | 5 |
| 11 | 5 | 32 | 5 | 61 | 5 |
| 12 | 5 | 33 | 5 | 62 | 5 |
| 13 | 5 | 34 | 4 | 63 | 5 |
| 14 | 5 | 35 | 4 | 64 | 5 |
| 15 | 5 | 36 | 4 | 65 | 5 |
| 16 | 5 | 37 | 4 | 66 | 5 |
| 17 | 5 | 38 | 4 | 67 | 5 |
| 18 | 5 | 42 | 5 | 68 | 5 |
| 19 | 5 | 45 | 5 | 69 | 5 |
| 20 | 5 | 46 | 5 | 70 | 5 |
| 21 | 5 | 47 | 5 | 71 | 5 |
| 22 | 5 | 48 | 5 | 72 | 5 |
| 23 | 5 | 49 | 5 | 74 | 5 |
| 24 | 5 | 50 | 5 | 75 | 5 |
| 25 | 5 | 51 | 5 | 76 | 5 |
| 26 | 5 | 52 | 5 | 78 | 5 |

Table 7 (contd)

| Compound No. | Effect | Compound No. | Effect | Compound No. | Effect |
|---|---|---|---|---|---|
| 79 | 5 | 98 | 5 | 114 | 5 |
| 80 | 5 | 99 | 5 | 124 | 4 |
| 81 | 5 | 100 | 5 | 125 | 5 |
| 85 | 5 | 101 | 5 | 126 | 5 |
| 86 | 5 | 103 | 5 | 127 | 5 |
| 87 | 5 | 104 | 5 | 128 | 5 |
| 90 | 5 | 105 | 5 | 129 | 5 |
| 91 | 5 | 108 | 5 | 130 | 4 |
| 93 | 5 | 110 | 5 | 133 | 5 |
| 94 | 4 | 111 | 5 | | |
| 95 | 4 | 112 | 5 | | |
| 96 | 4 | 113 | 5 | Reference G | 0 |

Test 7 Activity test against two-spotted spider mite egg

On filter papers soaked with water, kidney bean leaf strips of 20 mm in diameter were placed and 10 adult female two-spotted spider mites were inoculated and permitted to lay eggs for one day. Next, the adult female mites were removed, and each kidney leaf strip on which eggs were laid was dipped for 15 seconds into medicinal solutions prepared by forming the compounds shown in Table 1 into preparations similarly as in Example 17 and diluting with water containing a surfactant (0.01 %) to 300 ppm. After drying on air, the leaf strips were left to stand in a thermostat chamber at 25°C. On 8 days after the treatment, the numbers of non-hatched eggs were examined to determine the egg killing ratio. The acaricide ratio was evaluated as same as the insecticide ratio described in Test 1. The results are shown in Table 8.

Further, the compound represented by the foregoing formula [A] which is described in Japanese Provisional Patent Publication No. 130275/1986 was prepared and tested similarly to the above procedure, as reference H.

Table 8

| Compound No. | Effect | Compound No. | Effect | Compound No. | Effect |
|---|---|---|---|---|---|
| 3 | 4 | 25 | 5 | 49 | 5 |
| 6 | 5 | 26 | 5 | 50 | 5 |
| 8 | 5 | 27 | 5 | 51 | 5 |
| 9 | 5 | 28 | 5 | 52 | 5 |
| 10 | 5 | 29 | 5 | 53 | 5 |
| 11 | 5 | 30 | 5 | 55 | 5 |
| 12 | 5 | 31 | 5 | 56 | 5 |
| 13 | 5 | 32 | 5 | 57 | 5 |
| 14 | 5 | 33 | 5 | 58 | 5 |
| 15 | 5 | 34 | 4 | 61 | 5 |
| 16 | 5 | 36 | 5 | 62 | 5 |
| 17 | 5 | 37 | 5 | 63 | 5 |
| 18 | 5 | 38 | 5 | 64 | 5 |
| 19 | 5 | 41 | 4 | 65 | 5 |
| 20 | 5 | 42 | 5 | 66 | 5 |
| 21 | 5 | 45 | 5 | 67 | 5 |
| 22 | 5 | 46 | 5 | 68 | 5 |
| 23 | 5 | 47 | 5 | 69 | 5 |
| 24 | 5 | 48 | 5 | 70 | 5 |

Table 8 (Contd)

| Compound No. | Effect | Compound No. | Effect | Compound No. | Effect |
|---|---|---|---|---|---|
| 71 | 5 | 91 | 5 | 115 | 5 |
| 72 | 5 | 93 | 5 | 117 | 5 |
| 74 | 5 | 94 | 4 | 118 | 5 |
| 75 | 5 | 95 | 4 | 121 | 5 |
| 76 | 5 | 96 | 4 | 124 | 5 |
| 78 | 5 | 98 | 5 | 125 | 5 |
| 79 | 5 | 99 | 5 | 126 | 5 |
| 80 | 5 | 100 | 5 | 127 | 5 |
| 81 | 5 | 101 | 5 | 128 | 5 |
| 83 | 5 | 104 | 5 | 129 | 5 |
| 84 | 5 | 105 | 5 | 130 | 5 |
| 85 | 5 | 110 | 5 | 131 | 5 |
| 86 | 5 | 111 | 5 | 133 | 5 |
| 87 | 5 | 112 | 5 | | |
| 88 | 5 | 113 | 5 | | |
| 90 | 5 | 114 | 5 | Reference H | 0 |

Test 8 Activity test against tobacco cutworm

The compounds shown in Table 1 were formed into preparations similarly as in Example 16, diluted with water containing a surfactant (0.01 %) to 300 ppm. On the other hand, in plastic cups of 10 cm in diameter, leaves of soybean were laid, and 10 second-instar larvae of tobacco cutworm were provided for test on the leaves. The medicinal solution prepared was sprayed each in 5 ml in a spraying tower. Then, the leaf was left to stand in a thermostat chamber at 25 °C, and the numbers of alive and dead insects after 2 days were examined to determine the insecticide ratio. The results are shown in Table 9.

Further, the compound represented by the foregoing formula [A] which is described in Japanese Provisional Patent Publication No. 130275/1986 was prepared and tested similarly to the above procedure, as reference I.

Table 9

| Compound No. | Effect | Compound No. | Effect | Compound No. | Effect |
|---|---|---|---|---|---|
| 5 | 5 | 63 | 5 | 90 | 5 |
| 6 | 5 | 68 | 5 | 91 | 5 |
| 8 | 5 | 71 | 5 | 93 | 5 |
| 17 | 5 | 72 | 5 | 98 | 5 |
| 18 | 5 | 74 | 5 | 99 | 5 |
| 19 | 5 | 76 | 5 | 103 | 4 |
| 30 | 5 | 78 | 5 | 104 | 5 |
| 31 | 5 | 79 | 5 | 114 | 4 |
| 32 | 5 | 80 | 5 | 121 | 5 |
| 47 | 5 | 81 | 5 | 122 | 5 |
| 48 | 5 | 83 | 4 | 128 | 5 |
| 51 | 5 | 85 | 5 | 129 | 5 |
| 52 | 5 | 86 | 5 | 130 | 4 |
| 57 | 5 | 87 | 5 | 133 | 5 |
| 58 | 5 | 88 | 5 | Reference I | 0 |

Test 9 Control effect against downy mildew

In pots made of a plastic, soil medium was placed and cucumber seeds were planted. Cultivation was conducted in a hot house, and on the young seedlings with spreading of the first leaf was sprayed sufficient amounts of the medicinal solutions prepared by formimg the compounds shown in Table 1 into preparation similarly as described in Example 18 and diluting with water containing a surfactant (0.01 %) to 200 ppm. At 48 hours after spraying, a suspension of spores of *Pseudoperonospora cubensis* was inoculated by spraying and left to stand in a chamber at a temperature of 20 °C and a humidity of 100 % for 24 hours. The effect of medicine was judged by comparison of the lesion area on the day 10 after inoculation with the lesion area in the non-treated district by observation with eyes. Evaluation is shown at 6 ranks of 5 to 0, with no lesion being rated as 5, those with lesion area of 10 % or less as compared with the non-treated district as 4, those with about 20 % as 3, those with about 40 % as 2, those with about 60 % as 1, and those wholly afflicted with disease as 0. The results are shown in Table 10.

Further, the compound represented by the foregoing formula [A] which is described in Japanese Provisional Patent Publication No. 130275/1986 was prepared and tested similarly to the above procedure, as reference J.

Table 10

| Compound No. | Effect | Compound No. | Effect | Compound No. | Effect |
|---|---|---|---|---|---|
| 2 | 5 | 61 | 4 | 129 | 5 |
| 4 | 5 | 103 | 5 | 130 | 5 |
| 5 | 4 | 107 | 4 | 131 | 4 |
| 6 | 4 | 110 | 4 | 132 | |
| 8 | 4 | 111 | 5 | 133 | 5 |
| 9 | 5 | 113 | 5 | 134 | 4 |
| 15 | 5 | 114 | 5 | 135 | 5 |
| 34 | 4 | 115 | 5 | | |
| 35 | 5 | 128 | 5 | Reference J | 0 |

According to the present invention, novel pyridazinone derivatives having excellent insecticide effect, acaricide effect and fungicide effect can be provided.

**Claims**

1. A pyridazinone derivative represented by the formula:

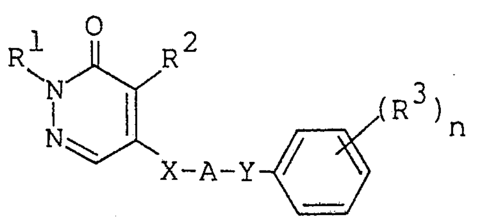

wherein $R^1$ represents a lower alkyl group; $R^2$ represents a halogen atom; $R^3$ represents a hydrogen atom, a halogen atom, a nitro group, a formyl group, a lower alkenyl group, a substituted or unsubstituted alkyl group having 1 to 10 carbon atoms, a lower alkoxy group, a substituted or unsubstituted 1,3-dioxolan-2-yl group, a lower alkoxyiminoalkyl group, an acyl group or a 1,3-thiooxolan-2-yl group; n represents an integer of 1 to 5; A represents a straight or branched alkylene group having 2 to 4 carbon atoms; and X and Y each independently represent an oxygen atom or a sulfur atom;
or an acid addition salt thereof.

2. A process for producing a pyridazinone derivative represented by the formula:

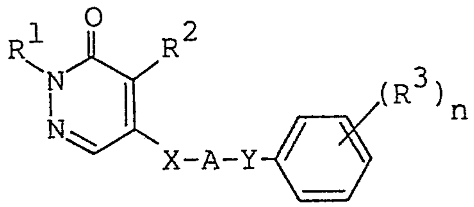

wherein $R^1$ represents a lower alkyl group; $R^2$ represents a halogen atom; $R^3$ represents a hydrogen atom, a halogen atom, a nitro group, a formyl group, a lower alkenyl group, a substituted or unsubstituted alkyl group having 1 to 10 carbon atoms, a lower alkoxy group, a substituted or unsubstituted 1,3-dioxolan-2-yl group, a lower alkoxyiminoalkyl group, an acyl group or a 1,3-thiooxolan-2-yl group; n represents an integer of 1 to 5; A represents a straight or branched alkylene group having 2 to 4 carbon atoms; and X and Y each independently represent an oxygen atom or a sulfur atom;
or an acid addition salt thereof, which comprises allowing a compound of the formula:

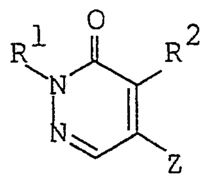

wherein $R^1$, $R^2$ and Z are as difined above,
to react with a compound of the formula:

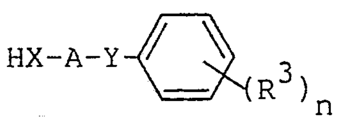

wherein $R^3$, n, A, X and Y are as defined above.

3. A process for producing a pyridazinone derivative represented by the formula:

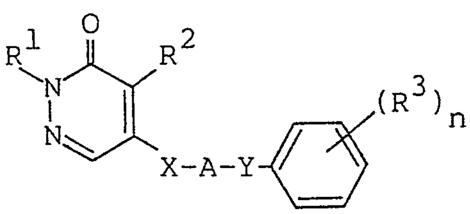

wherein $R^1$ represents a lower alkyl group; $R^2$ represents a halogen atom; $R^3$ represents a hydrogen atom, a halogen atom, a nitro group, a formyl group, a lower alkenyl group, a substituted or unsubstituted alkyl group having 1 to 10 carbon atoms, a lower alkoxy group, a substituted or unsubstituted 1,3-dioxolan-2-yl group, a lower alkoxyiminoalkyl group, an acyl group or a 1,3-thiooxolan-2-yl group; n represents an integer of 1 to 5; A represents a straight or branched alkylene group having 2 to 4 carbon atoms; and X and Y each independently represent an oxygen atom or a sulfur atom;
or an acid addition salt thereof, which comprises allowing a compound of the formula:

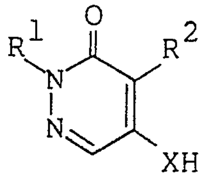

wherein $R^1$, $R^2$ and X are as difined above,
to react with a compound of the formula:

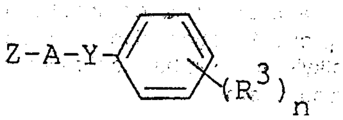

wherein $R^3$, n, A, Y and Z are as defined above.

4. An insecticide comprising as the active ingredient a pyridazinone derivative represented by the formula:

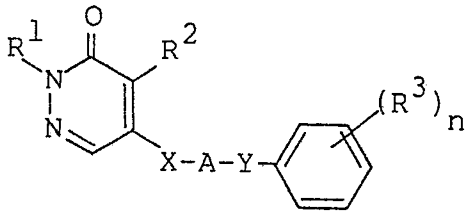

wherein $R^1$ represents a lower alkyl group; $R^2$ represents a halogen atom; $R^3$ represents a hydrogen atom, a halogen atom, a nitro group, a formyl group, a lower alkenyl group, a substituted or unsubstituted alkyl group having 1 to 10 carbon atoms, a lower alkoxy group, a substituted or unsubstituted 1,3-dioxolan-2-yl group, a lower alkoxyiminoalkyl group, an acyl group or a 1,3-thiooxolan-2-yl group; n represents an integer of 1 to 5; A represents a straight or branched alkylene group having 2 to 4 carbon atoms; and X and Y each independently represent an oxygen atom or a sulfur atom;
or an acid addition salt thereof.

5. An acaricide comprising as the active ingredient a pyridazinone derivative represented by the formula:

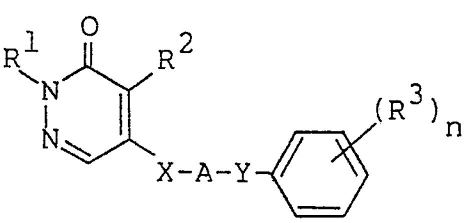

wherein $R^1$ represents a lower alkyl group; $R^2$ represents a halogen atom; $R^3$ represents a hydrogen atom, a halogen atom, a nitro group, a formyl group, a lower alkenyl group, a substituted or unsubstituted alkyl group having 1 to 10 carbon atoms, a lower alkoxy group, a substituted or unsubstituted 1,3-dioxolan-2-yl group, a lower alkoxyiminoalkyl group, an acyl group or a 1,3-thiooxolan-2-yl group; n represents an integer of 1 to 5; A represents a straight or branched alkylene group having 2 to 4 carbon atoms; and X and Y each independently represent an oxygen atom or a sulfur atom;
or an acid addition salt thereof.

6. A fungicide comprising as the active ingredient a pyridazinone derivative represented by the formula:

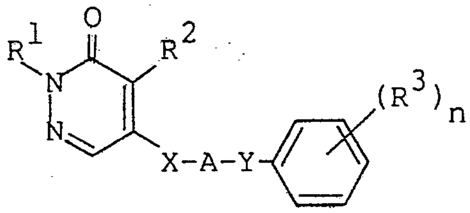

wherein $R^1$ represents a lower alkyl group; $R^2$ represents a halogen atom; $R^3$ represents a hydrogen atom, a halogen atom, a nitro group, a formyl group, a lower alkenyl group, a substituted or unsubstituted alkyl group having 1 to 10 carbon atoms, a lower alkoxy group, a substituted or unsubstituted 1,3-dioxolan-2-yl group, a lower alkoxyiminoalkyl group, an acyl group or a 1,3-thiooxolan-2-yl group; n represents an integer of 1 to 5; A represents a straight or branched alkylene group having 2 to 4 carbon atoms; and X and Y each independently represent an oxygen atom or a sulfur atom;
an acid addition salt thereof.

Claims for the following contracting State: ES

1. A process for producing a pyridazinone derivative represented by the formula:

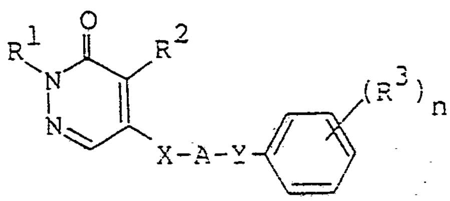

wherein $R^1$ represents a lower alkyl group; $R^2$ represents a halogen atom; $R^3$ represents a hydrogen atom, a halogen atom, a nitro group, a formyl group, a lower alkenyl group, a substituted or unsubstituted alkyl group having 1 to 10 carbon atoms, a lower alkoxy group, a substituted or unsubstituted 1,3-dioxolan-2-yl group, a lower alkoxyiminoalkyl group, an acyl group or a 1,3-thiooxolan-2-yl group; n represents an integer of 1 to 5; A represents a straight or branched alkylene group having 2 to 4 carbon atoms; and X and Y each independently represent an oxygen atom or a sulfur atom;
or an acid addition salt thereof, which comprises allowing a compound of the formula:

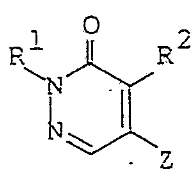

wherein $R^1$, $R^2$ and Z are as defined above,
to react with a compound of the formula:

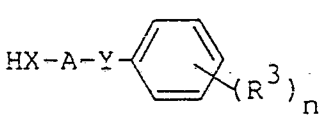

wherein $R^3$, n, A, X and Y are as defined above.

2. A process for producing a pyridazinone derivative represented by the formula:

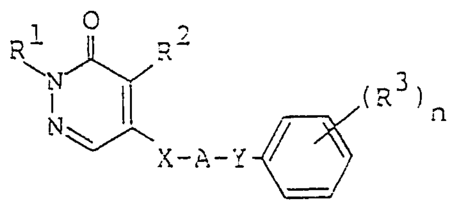

wherein $R^1$ represents a lower alkyl group; $R^2$ represents a halogen atom; $R^3$ represents a hydrogen atom, a halogen atom, a nitro group, a formyl group, a lower alkenyl group, a substituted or unsubstituted alkyl group having 1 to 10 carbon atoms, a lower alkoxy group, a substituted or unsubstituted 1, 3-dioxolan-2-yl group, a lower alkoxyiminoalkyl group, an acyl group or a 1,3-thiooxolan-2-yl group; n represents an integer of 1 to 5; A represents a straight or branched alkylene group having 2 to 4 carbon atoms; and X and Y each independently represent an oxygen atom or a sulfur atom:
or an acid addition salt thereof, which comprises allowing a compound of the formula:

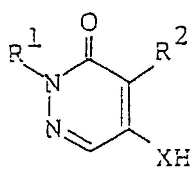

wherein $R^1$, $R^2$ and X are as defined above,
to react with a compound of the formula:

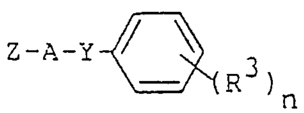

wherein $R^3$, n, A, Y and Z are as defined above.